# EUROPEAN PATENT SPECIFICATION

(11) **EP 4 303 677 B1**
(45) Date of publication and mention of the grant of the patent: **02.12.2026**
(21) Application number: 23175983.8
(22) Date of filing: 30.05.2023
(51) Int. Cl.: G05B 19/418, B29C 70/54, G05B 13/02, G05B 13/04

(54) **PROPERTY PREDICTION FOR UNCONSOLIDATED COMPOSITE MATERIALS**
VORHERSAGE VON EIGENSCHAFTEN FÜR UNVERFESTIGTE VERBUNDWERKSTOFFE
PRÉDICTION DE PROPRIÉTÉS POUR MATÉRIAUX COMPOSITES NON CONSOLIDÉS

(30) Priority: 08.07.2022 US 202217811433
(43) Date of publication of application: 10.01.2024
(73) Proprietor: The Boeing Company, Arlington, VA 22202 (US)
(72) Inventor: STEWART, Tiffany Allison, Arlington, 22202 (US); SHAHAN, David Williamson, Arlington, 22202 (US); HENRY, Christopher, Arlington, 22202 (US); HUNDLEY, Jacob Michael, Arlington, 22202 (US); MCKNIGHT, Geoffrey, Arlington, 22202 (US); BAUER, Andrew L, Arlington, 22202 (US); LANPHERE GROSS, Gwen Marie, Arlington, 22202 (US); ROGALSKI, Mark Eugene, Arlington, 22202 (US)
(74) Representative: Boult Wade Tennant LLP

(56) References cited:
- EP-A1- 3 751 367
- WO-A1-2016/044415
- US-A1- 2006 071 668
- US-A1- 2016 299 085
- US-A1- 2020 264 335
- US-A1- 2021 252 744

## Description

### BACKGROUND INFORMATION

### 1. Field:

The present disclosure relates generally to manufacturing composite materials and in particular, to manufacturing uncured composite materials. Still more particularly, the present disclosure relates to a method, apparatus, system, and computer program product for predicting properties for uncured composite materials during the manufacturing of the uncured composite materials.

### 2. Background:

Aircraft are being designed and manufactured with greater and greater percentages of composite materials. Composite materials are used in aircraft to decrease the weight of the aircraft and improve aero efficiency through design. This decreased weight improves performance features such as increased payload capacities and fuel efficiencies. Further, composite materials provide longer service life for various components in an aircraft.

Composite materials are tough, light-weight materials created by combining two or more functional components. For example, a composite material may include reinforcing fibers bound in a polymer resin matrix. The fibers may be unidirectional or may take the form of a woven cloth or fabric. The fibers and resins are arranged and cured to form a composite material.

Further, using composite materials to create aerospace composite structures potentially allows for portions of an aircraft to be manufactured in larger pieces or sections. For example, a fuselage in an aircraft may be created in cylindrical sections and then assembled to form the fuselage of the aircraft. Other examples include, without limitation, wing sections joined to form a wing or stabilizer sections joined to form a stabilizer.

In manufacturing composite structures, layers of composite material are typically laid up on a tool. The layers may be comprised of fibers in sheets. These sheets may take the form of fabrics, tape, tows, or other suitable forms. In some cases, resin may be infused or preimpregnated into the sheets. These types of sheets are commonly referred to as prepreg. Prepreg can be manufactured in long sheets that are stored in rolls until the prepreg is used to manufacture composite structures.

Different layers of prepreg may be laid up in different orientations and different numbers of layers may be used depending on the thickness of the composite structure being manufactured. These layers may be laid up by hand or using automated lamination equipment such as a tape laminating machine or a fiber placement system.

After the different layers of composite material have been laid up on the tool, the layers of composite material may be consolidated and cured upon exposure to temperature and pressure, thus forming the final composite structure.

The quality of the final composite structure can be determined using testing, including non-destructive testing. Further, components used to manufacture a composite structure can also be monitored and tested for quality purposes. Maintaining a desired quality for composite components can increase the quality of the final composite structures. For example, prepreg can be inspected to determine whether the thickness is within specified tolerances. The quality testing of composite components, such as prepreg components, can be more time consuming than desired.

Therefore, it would be desirable to have a method and apparatus that take into account at least some of the issues discussed above, as well as other possible issues. For example, it would be desirable to have a method, apparatus, system, and computer program product that overcome a technical problem with quality testing of prepreg components.

US 2016/299085 A1, in accordance with its abstract, states that there is a method of controlling a process for the manufacture of a multicomponent sheet material having a desired pre-determined parameter comprising applying electromagnetic energy to interact with the sheet material whereby the interaction modifies the applied energy, detecting the modified energy, comparing the modified energy or data derived from it with data relating to the pre-determined parameter and modifying at least one step of the process whereby the data relating to the modified energy is modified towards the data relating to the predetermined parameter.

US 2021/252744 A1, in accordance with its abstract, states that a resin supply unit of a manufacturing apparatus supplies a resin to a roller surface of an impregnated roller which is capable of rotating at a constant speed. A transport mechanism brings the fiber bundle into contact with the resin on the roller surface and thereby forms a tow prepreg while the fiber bundle is being transported. A fineness acquisition unit acquires as a fineness acquisition value a fineness, which is defined by a mass per unit length of the fiber bundle during conveyance thereof before being brought into contact with the resin. A resin supply amount control unit controls the resin supply amount based on the fineness acquisition value, in a manner so that a resin content of the tow prepreg becomes a target resin content.

EP 3 751 367 A1, in accordance with its abstract, states that a method of additive manufacturing machine (AMM) build process control includes obtaining AMM machine and process parameter settings, accessing sensor data for monitored physical conditions in the AMM, calculating a difference between expected AMM physical conditions and elements of the monitored conditions, providing the machine and process parameter settings, monitored conditions, and differences to one or more material property prediction models, computing a predicted value or range for the monitored conditions, comparing the predicted value or range to a predetermined target range, based on a determination that predicted value(s) are within the predetermined range, maintaining the machine and process parameter settings, or based on a determination that one or more of the predicted value(s) is outside the predetermined range, generating commands to compensate the machine and process parameter settings, and repeating the closed feedback loop at intervals of time during the build process. A system and a non-transitory medium are also disclosed.

US 2020/0264335 A1, in accordance with its abstract, states that methods, systems, and non-transitory computer readable medium are described for sensor metrology data integration. A method includes receiving sets of sensor data and sets of metrology data. Each set of sensor data includes corresponding sensor values associated with producing corresponding product by manufacturing equipment and a corresponding sensor data identifier. Each set of metrology data includes corresponding metrology values associated with the corresponding product manufactured by the manufacturing equipment and a corresponding metrology data identifier. The method further includes determining common portions between each corresponding sensor data identifier and each corresponding metrology data identifier. The method further includes, for each of the sensor-metrology matches, generating a corresponding set of aggregated sensor-metrology data and storing the sets of aggregated sensor-metrology data to train a machine learning model. The trained machine learning model is capable of generating one or more outputs for performing a corrective action associated with the manufacturing equipment.

### SUMMARY

There is described herein a computer-implemented method for manufacturing an unconsolidated composite material, the method comprising: receiving sensor data from a sensor system for a composite material manufacturing system, wherein the sensor data is about at least one of the unconsolidated composite material or the composite material manufacturing system during the operation of composite material manufacturing system and is received during manufacturing of the unconsolidated composite material by the composite material manufacturing system; determining a set of predicted properties for a number of portions of the unconsolidated composite material as completed from manufacturing by the composite material manufacturing system using the sensor data and a machine learning model, wherein the machine learning model is trained to predict properties for the unconsolidated composite material using sensor data, the machine learning model has been trained using a training dataset comprising historical sensor data generated from previously manufacturing the unconsolidated composite material and labels in the form of historical properties, and the historical properties are determined from measurements made of unconsolidated composite material at different locations in the composite material manufacturing system, and include properties of unconsolidated composite material when manufacturing of unconsolidated composite material has been completed; and identifying a quality level for the number of portions of the unconsolidated composite material based on the set of predicted properties for the number of portions of the unconsolidated composite material.

There is also described herein a composite material management system comprising: a computer system including a composite material manager, wherein the composite material manager is programmed to perform the method described above.

There is also described herein, a computer program comprising computer readable program instructions that, when executed by one or more computer processors of a computer system, cause the computer system to perform the method described above. There is also described herein a computer readable storage medium having stored thereon such a computer program.

Of the appended independent claims, claim 1 defines a method for manufacturing an unconsolidated composite material, claim 11 defines a composite material management system, claim 13 defines a composite material manufacturing system, claim 14 defines a computer program and claim 15 defines a computer-readable medium. The dependent claims describe optional features.

### BRIEF DESCRIPTION OF THE DRAWINGS

The novel features believed characteristic of the illustrative examples are set forth in the appended claims. The illustrative examples, however, as well as a preferred mode of use, further objectives and features thereof, will best be understood by reference to the following detailed description of an illustrative example of the present disclosure when read in conjunction with the accompanying drawings, wherein:
**Figure 1** is a pictorial representation of a network of data processing systems in which illustrative examples may be implemented;
**Figure 2** is a block diagram of a composite manufacturing environment in accordance with an illustrative example;
**Figure 3** is an illustration of a block diagram of actions that can be taken using predicted properties for portions of an unconsolidated composite material in accordance with an illustrative example;
**Figure 4** is an illustration of a manufacturing process flow for manufacturing unconsolidated composite materials in accordance with an illustrative example
**Figure 5** is an illustration of a composite material manufacturing system configured for performing filming process in accordance with an illustrative example;
**Figure 6** is an illustration of a composite material manufacturing system configured for performing a prepreg production process in accordance with an illustrative example;
**Figure 7****,** an illustration of a block diagram of a sensor location in a composite material manufacturing system in accordance with an illustrative example;
**Figure 8** is an illustration of the side view of a portion of a composite material manufacturing system in accordance with an illustrative example;
**Figure 9** is an illustration of the top view of a portion of a composite material manufacturing system in accordance with an illustrative example;
**Figure 10** is an illustration of a flowchart of a process for identifying quality levels for portions of an unconsolidated composite material in accordance with an illustrative example;
**Figure 11** is an illustration of a flowchart of a process for marking an unconsolidated composite material in accordance with an illustrative example;
**Figure 12** is an illustration of a flowchart of a process for marking an out-of-tolerance portion of an unconsolidated composite material as unusable in accordance with an illustrative example;
**Figure 13** is an illustration of a flowchart of a process for marking an in-tolerance portion of an unconsolidated composite material as usable in accordance with an illustrative example;
**Figure 14** is an illustration of a flowchart of a process for marking a portion of an unconsolidated composite material with a quality level in accordance with an illustrative example;
**Figure 15** is an illustration of a flowchart of a process for recording a quality level for portions of an unconsolidated composite material in accordance with an illustrative example;
**Figure 16** is an illustration of a flowchart of a process for determining predicted properties for a portion of an unconsolidated composite material in accordance with an illustrative example;
**Figure 17** is an illustration of a flowchart of a process for training a machine learning model to predict properties for an unconsolidated composite material prior to completing fabrication of the unconsolidated composite material in accordance with an illustrative example;
**Figure 18** is an illustration of a flowchart of a process for performing a corrective action for portions of an unconsolidated composite material in accordance with an illustrative example;
**Figure 19** is an illustration of a flowchart of a process for performing a corrective action for out-of-tolerance portions of an unconsolidated composite material in accordance with an illustrative example;
**Figure 20** is an illustration of a flowchart of a process for adjusting parameters for out-of-tolerance portions of an unconsolidated composite material in accordance with an illustrative example;
**Figure 21** is an illustration of a flowchart of a process for adjusting parameters for in-tolerance portions of an unconsolidated composite material in accordance with an illustrative example;
**Figure 22** is an illustration of a flowchart of a process for adjusting parameters such that portions of an unconsolidated composite material are within tolerance in accordance with an illustrative example;
**Figure 23** is an illustration of a flowchart of a process for adjusting parameters for a subsequent portion of an unconsolidated composite material in accordance with an illustrative example;
**Figure 24** is an illustration of a flowchart of a process for changing raw materials in accordance with an illustrative example;
**Figure 25** is an illustration of a flowchart of a process for determining predicted properties of an unconsolidated composite material in accordance with an illustrative example;
**Figure 26** is an illustration of a block diagram of a data processing system in accordance with an illustrative example;
**Figure 27** is an illustration of an aircraft manufacturing and service method in accordance with an illustrative example;
**Figure 28** is an illustration of a block diagram of an aircraft in which an illustrative example may be implemented; and
**Figure 29** is an illustration of a block diagram of a product management system in accordance with an illustrative example.

### DETAILED DESCRIPTION

The illustrative examples recognize and take into account one or more different considerations as described below. For example, current testing of unconsolidated composite components, such as a prepreg roll manufactured by carbon fiber prepreg coating line, can be performed using a sample from the prepreg roll. If the sample does not meet a desired quality level, the entire prepreg roll is discarded or recycled. However, the entire prepreg roll may not have an undesired quality level. Only a portion of the prepreg roll may not meet tolerances for the desired quality level. Current techniques do not enable identifying which portions of the prepreg roll may have properties that are out of tolerance without testing multiple portions of the entire prepreg roll. Testing multiple portions of the entire prepreg roll is time consuming and inefficient.

Also determining the cause for the prepreg roll to have properties with an undesired quality level cannot currently be performed during the manufacturing process. Instead, an analysis is performed after manufacturing the prepreg roll and testing a sample of the prepreg roll indicates the sample does not have the desired quality level. Further, when the cause can be determined, adjustments to the carbon fiber prepreg coating line can be made to ensure future prepreg rolls have a desired quality level. The time needed for the determination may not be possible before more prepreg rolls have been completed and one or more have been determined to not have a desired quality level. As a result, one solution is to halt manufacturing of prepreg rolls using the carbon fiber prepreg coating line that manufactured the prepreg roll until the cause can be determined and adjustments can be made to the carbon fiber prepreg coating line. This unavailability to the carbon fiber prepreg coating line reduces the capacity to manufacture prepreg rolls.

The illustrative examples further recognize and take into account that it would be desirable to have an ability to determine the characteristics of prepreg materials. The manufacturing process can be at least one of resin mixing, resin filming, a resin bath operation, or other operations performed to produce prepreg during the manufacturing process while the prepreg is being manufactured. The different types of sensor data obtained from different locations in the carbon fiber prepreg coating line in real time can be used to predict the characteristics of the prepreg while the prepreg is still being manufactured. This information can be used to determine which portions of the prepreg roll have characteristics that are in tolerance and other portions of the prepreg roll that are out of tolerance. This type of determination of characteristics for portions of the prepreg being manufactured can be in real time in a manner that allows marking or recording which portions are in tolerance or out of tolerance. As a result, when some portions of the prepreg roll are out of tolerance, the portions recorded or marked to not be used while the other portion of the prepreg rolls can still be used. As a result, the amount of prepreg discarded or recycled can be reduced

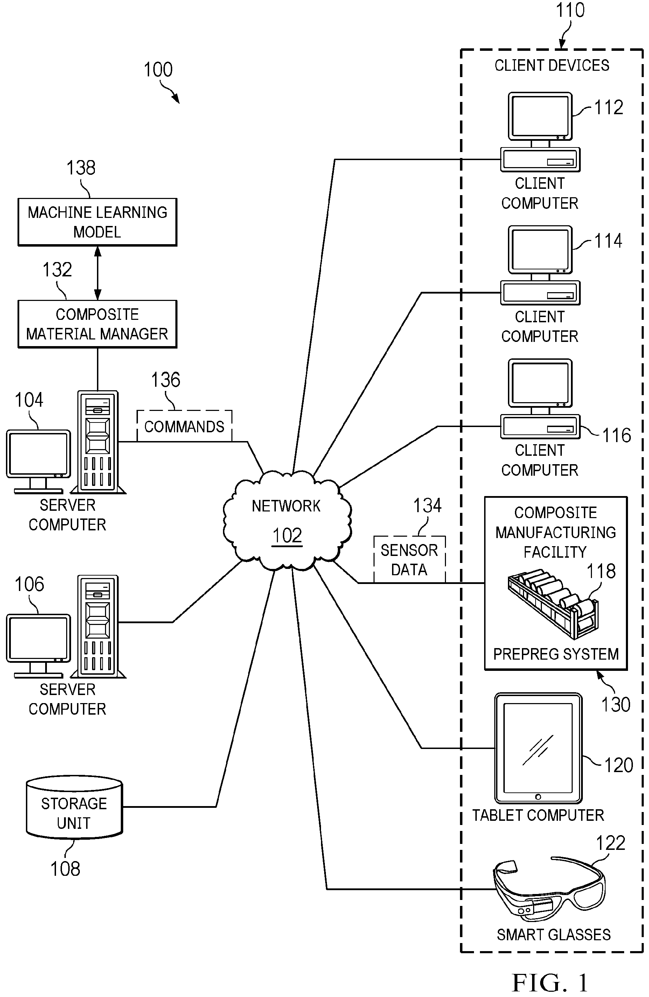

With reference now to the figures and, in particular, with reference to **Figure 1****,** a pictorial representation of a network of data processing systems is depicted in which illustrative examples may be implemented. Network data processing system **100** is a network of computers in which the illustrative examples may be implemented. Network data processing system **100** contains network **102,** which is the medium used to provide communications links between various devices and computers connected together within network data processing system **100.** Network **102** may include connections, such as wire, wireless communication links, or fiber optic cables.

In some examples including the depicted example, server computer **104** and server computer **106** connect to network **102** along with storage unit **108.** In addition, client devices **110** connect to network **102.** As depicted, client devices **110** include client computer **112,** client computer **114,** and client computer **116.** Client devices **110** can be, for example, computers, workstations, or network computers. In some examples including the depicted example, server computer **104** provides information, such as boot files, operating system images, and applications to client devices **110.** Further, client devices **110** can also include other types of client devices such as prepreg system **118,** tablet computer **120,** and smart glasses **122.** In this and selected other examples, server computer **104,** server computer **106,** storage unit **108,** and client devices **110** are network devices that connect to network **102** in which network **102** is the communications media for these network devices. Some or all of client devices **110** may form an Internet of Things (IoT) in which these physical devices can connect to network **102** and exchange information with each other over network **102.**

Client devices **110** are clients to server computer **104** in this and selected other examples. Network data processing system **100** may include additional server computers, client computers, and other devices not shown. Client devices **110** connect to network **102** utilizing at least one of wired, optical fiber, or wireless connections.

Program instructions located in network data processing system **100** can be stored on a computer-recordable storage media and downloaded to a data processing system or other device for use. For example, program instructions can be stored on a computer-recordable storage media on server computer **104** and downloaded to client devices **110** over network **102** for use on client devices **110.**

In some examples including the depicted example, network data processing system **100** is the Internet with network **102** representing a worldwide collection of networks and gateways that use the Transmission Control Protocol/Internet Protocol (TCP/IP) suite of protocols to communicate with one another. At the heart of the Internet is a backbone of high-speed data communication lines between major nodes or host computers consisting of thousands of commercial, governmental, educational, and other computer systems that route data and messages. Of course, network data processing system **100** also may be implemented using a number of different types of networks. For example, network **102** can be comprised of at least one of the Internet, an intranet, a local area network (LAN), a metropolitan area network (MAN), or a wide area network (WAN). **Figure 1** is intended as an example, and not as an architectural limitation for the different illustrative examples.

As used herein, "a number of" when used with reference to items, means one or more items. For example, "a number of different types of networks" is one or more different types of networks.

Further, the phrase "at least one of," when used with a list of items, means different combinations of one or more of the listed items can be used, and only one of each item in the list may be needed. In other words, "at least one of" means any combination of items and number of items may be used from the list, but not all of the items in the list are required. The item can be a particular object, a thing, or a category.

For example, without limitation, "at least one of item A, item B, or item C" may include item A, item A and item B, or item B. This example also may include item A, item B, and item C or item B and item C. Of course, any combinations of these items can be present. In some illustrative examples, "at least one of" can be, for example, without limitation, two of item A; one of item B; and ten of item C; four of item B and seven of item C; or other suitable combinations.

In this and selected other examples, prepreg system **118** is located in composite manufacturing facility **130.** This location manufactures unconsolidated composite materials such as prepreg using prepreg system **118.** As depicted, composite material manager **132** at server computer **104** can communicate with prepreg system **118** at composite manufacturing facility **130.** This communication can include the exchange of information selected from at least one of sensor data **134,** commands **136,** or other types of information that can be sent over network **102.**

In this and selected other examples, composite material manager **132** can receive sensor data **134** from a sensor system for prepreg system **118.** The sensor system can be located in at least one of prepreg system **118** or another location in composite manufacturing facility **130.**

In this and selected other examples, sensor data **134** is received in real time from prepreg system **118.** In other words, receiving sensor data **134** in real-time means that sensor data **134** is received as prepreg system **118** operates to manufacture prepreg. When sensor data **134** is sent in real time, sensor data **134** is sent as quickly as possible without potential delays. In analyzing sensor data **134** in real time, composite material manager **132** can process and analyze sensor data **134** as sensor data **134** is received.

As depicted, sensor data **134** can be analyzed by composite material manager **132** to determine a prediction of properties for the prepreg when manufacturing of the prepreg is completed. This prediction of properties is made while the prepreg is being manufactured by prepreg system **118.** The predicted properties are properties for a number of portions of the prepreg being manufactured within prepreg system **118.** These properties can include, for example, prepreg thickness, areal weight, and other characteristics of the completed prepreg. In other words, composite material manager **132** can perform real-time predicting of characteristics of the prepreg being manufactured by prepreg system **118** before manufacturing of the prepreg has been completed. Based on the predicted properties, composite material manager **132** can identify which of the portions of the prepreg are within a tolerance for use in manufacturing a composite part. Based on the set properties predicted for the prepreg, composite material manager **132** can record one or more portions that are within tolerance as well as portions that may be out of tolerance for use in manufacturing a composite part. Further, composite material manager **132** can determine how long the portions will remain within tolerance. In some cases, the unconsolidated composite material may have a shelf life which may vary depending on the properties predicted for the prepreg.

Additionally, with knowing the predicted properties for a portion of the prepreg, composite material manager **132** performs corrective actions based on the quality level determined for the portion of the unconsolidated composite material from the prediction of the properties of unconsolidated composite materials made prior manufacturing operations being completed when the portion is completed product in the unconsolidated composite material.

For example, composite material manager **132** can determine what adjustments are needed to prepreg system **118** such that further processing of the portion of the prepreg results in that portion being within tolerance or having a desired quality level. With this determination, composite material manager **132** can send commands **136** to prepreg system **118** to make the adjustments.

Using commands **136,** these adjustments can be made by composite material manager **132** while the portion of the prepreg is still being manufactured. These adjustments can be made to portions of prepreg system **118** that have not yet processed the portion of the prepreg. With these adjustments, additional sensor data can be received to determine whether the action performed has changed the portions of the prepreg to have a desired quality level. In this manner, composite material manager **132** can manage the manufacturing of the unconsolidated composite material in manner that can increase the portions of the unconsolidated composite material that will meet a desired quality level and reduce discarding portions unconsolidated composite material.

In this and other examples, this prediction can be made using machine learning model **138.** In this and selected other examples, machine learning model **138** has been trained using historical sensor data generated from previously manufacturing prepreg by prepreg system **118.**

Illustration of network data processing system **100 in** **Figure 1** is not meant to limit the manner in which other illustrative examples can be implemented. For example, composite material manager **132** can be located in another location other than in server computer **104.** For example, composite material manager **132** can be located at client computer **112,** in prepreg system **118,** or another computer located at composite manufacturing facility **130.** In other illustrative examples, additional prepreg systems may be located at composite manufacturing facility **130** or other locations that can be managed by composite material manager **132.**

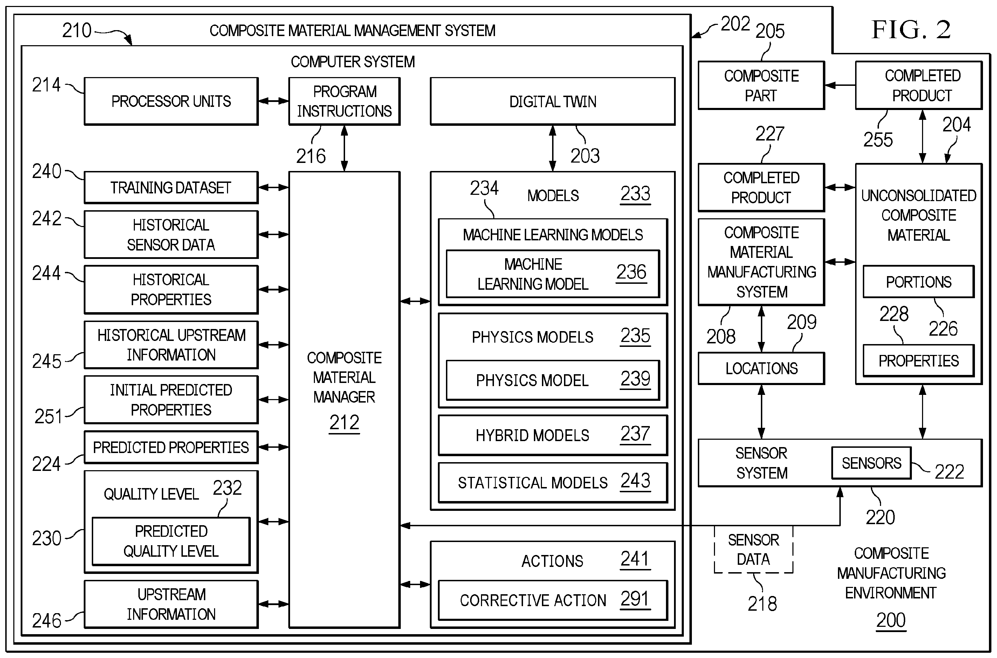

With reference now to **Figure 2****,** a block diagram of a composite manufacturing environment is depicted in accordance with an illustrative example. In this and selected other examples, composite manufacturing environment **200** includes components that can be implemented in hardware such as the hardware shown in network data processing system **100 in** **Figure 1****.**

In this and selected other examples, composite material management system **202** operates to manage the manufacturing of unconsolidated composite material **204** for use in manufacturing composite part **205.**

Unconsolidated composite material **204** is a composite material that has not been processed to form composite part **205.** For example, processing such as at least one of pressure, heating, or curing has not been applied to unconsolidated composite material **204.** In other words, unconsolidated composite material **204** has not been processed in a manner that forms the final composite part.

Unconsolidated composite material **204** can be selected from at least one of a prepreg, an aerospace-grade prepreg, a thermoset prepreg, a thermoplastic prepreg, a woven fabric prepreg, a fiber tow prepreg, a tow prepreg, unidirectional tape prepreg, a resin coated film, non-crimp fabric, a resin mix, or other suitable unconsolidated composite materials. Fibers in the unconsolidated composite material **204** can take a number of different forms. For example, unconsolidated composite material **204** such as fibers and fabric can be selected from at least one of glass fibers, carbon fibers, Kevlar fibers, para-aramid fibers, hybrid fibers, carbon fabric with a glass yarn, carbon fiber fabric with an interwoven wire, boron silicate fibers, or other suitable type of materials. Composite part **205** can be, for example, a fuselage section, skin panel, a wing, a rib, a door, or other suitable types of parts.

In some other examples, unconsolidated composite material **204** can be used to manufacture another unconsolidated composite material. For example, unconsolidated composite material **204** can be a filmed paper. This filmed paper can be used to manufacture another unconsolidated composite material such as prepreg.

For example, composite material management system **202** can control the operation of composite material manufacturing system **206** to manufacture unconsolidated composite material **204.** As depicted, composite material manufacturing system **206** can take a number of forms. For example, composite material manufacturing system **206** can be selected from at least one of a carbon fiber prepreg coating line, a reinforcing fiber resin impregnation line, a resin mix process system, a resin filming line, a prepreg system, a slitting process system, and other suitable types of composite material manufacturing systems.

As depicted, composite material management system **202** comprises computer system **210** and composite material manager **212.** Composite material manager **212** is located in computer system **210.**

Composite material manager **212** can be implemented in software, hardware, firmware or a combination thereof. When software is used, the operations performed by composite material manager **212** can be implemented in program instructions configured to run on hardware, such as a processor unit. When firmware is used, the operations performed by composite material manager **212** can be implemented in program instructions and data and stored in persistent memory to run on a processor unit. When hardware is employed, the hardware can include circuits that operate to perform the operations in composite material manager **212.**

In the illustrative examples, the hardware can take a form selected from at least one of a circuit system, an integrated circuit, an application specific integrated circuit (ASIC), a programmable logic device, or some other suitable type of hardware configured to perform a number of operations. With a programmable logic device, the device can be configured to perform the number of operations. The device can be reconfigured at a later time or can be permanently configured to perform the number of operations. Programmable logic devices include, for example, a programmable logic array, a programmable array logic, a field programmable logic array, a field programmable gate array, and other suitable hardware devices. Additionally, the processes can be implemented in organic components integrated with inorganic components and can be comprised entirely of organic components excluding a human being. For example, the processes can be implemented as circuits in organic semiconductors.

Computer system **210** is a physical hardware system and includes one or more data processing systems. When more than one data processing system is present in computer system **210,** those data processing systems are in communication with each other using a communications medium. The communications medium can be a network. The data processing systems can be selected from at least one of a computer, a server computer, a tablet computer, or some other suitable data processing system.

As depicted, computer system **210** includes a number of processor units **214** that are capable of executing program instructions **216** implementing processes in the illustrative examples. As used herein a processor unit in the number of processor units **214** is a hardware device and is comprised of hardware circuits such as those on an integrated circuit that respond and process instructions and program code that operate a computer. When a number of processor units **214** execute program instructions **216** for a process, the number of processor units **214** is one or more processor units that can be on the same computer or on different computers. In other words, the process can be distributed between processor units on the same or different computers in a computer system. Further, the number of processor units **214** can be of the same type or different type of processor units. For example, a number of processor units can be selected from at least one of a single core processor, a dual-core processor, a multi-processor core, a general-purpose central processing unit (CPU), a graphics processing unit (GPU), a digital signal processor (DSP), or some other type of processor unit.

In this and selected other examples, composite material manager **212** is configured to receive sensor data **218** from sensor system **220** for composite material manufacturing system **208.** Sensor data **218** can take a number of different forms. For example, sensor data **218** can be comprised from at least one of a component temperature, a unconsolidated composite material temperature, an ambient temperature, a humidity, a roller speed, a speed of the unconsolidated composite material, a pressure between a pair of rollers, a gap between the pair of rollers, an unconsolidated composite material thickness, a Fourier transform infrared (FTIR) spectra, Raman spectroscopy data, spectroscopy data, a material width, a fiber web width, a material areal weight, a fiber areal weight, a resin viscosity, an electrical measurement across a dimension of the unconsolidated composite material, a voltage across the dimension of the unconsolidated composite material, an electromagnetic measurement across the dimension of the unconsolidated composite material, an acoustic measurement across the dimension of the unconsolidated composite material, a tension, an image of a prepreg at a location in the composite material manufacturing system.

In this and selected other examples, sensor system **220** for composite material manufacturing system **208** can be considered part of composite material management system **202,** composite material manufacturing system **208,** or both. Sensor system **220** can operate to generate sensor data **218** about at least one of unconsolidated composite material **204** or composite material manufacturing system **208** during the operation of composite material manufacturing system **208.** Sensor data **218** can be information about unconsolidated composite material **204** being manufactured by composite material manufacturing system **208.** Sensor data **218** is received during manufacturing of unconsolidated composite material **204** by composite material manufacturing system **208.** Sensor data **218** is generated by sensor system **220** in real time and is sent to composite material manager **212** in real time.

Sensor system **220** comprises sensors **222.** As depicted, sensors **222** are in locations **209** relative to composite material manufacturing system **208.** Locations **209** can be on, in, or proximate to composite material manufacturing system **208.** In this example, sensors **222** can be selected from at least one of a temperature sensor, a camera, infrared camera, a thermal camera, line scan camera, a tension sensor, a density sensor, a drop gauge, an infrared sensor, a noncontact laser distance sensor, a beta gauge, an speed sensor, a Fourier transform infrared (FTIR) spectrometer, Raman spectrometer, a force detector, a linear variable differential transformer (LVDT) sensor, a rotary third differential transformer (RVDT) sensor, a thickness sensor, a gap width sensor, a viscometer, a light reflecting sensor, an electrical conductivity sensor, an electrical resistance sensor, a capacitance sensor, an electromagnetic sensor, an acoustic sensor, a humidity sensor, a laser Doppler sensor, or other suitable sensors.

As depicted, composite material manager **212** determines a set of predicted properties **224** for a number of portions **226** of unconsolidated composite material **204** as completed from manufacturing by composite material manufacturing system **208** using sensor data **218.**

The number of portions **226** of unconsolidated composite material **204** can be a part or all of portions **226** of unconsolidated composite material **204.** In some examples including this depicted example, unconsolidated composite material **204** as completed from manufacturing by composite material manufacturing system **208** forms completed product **255,** which can be used to manufacture composite part **205.** The number of portions **226** can be sections of unconsolidated composite material **204.** For example, the number of portions **226** can be pieces or sections of prepreg that form a prepreg roll. These portions can have the same or different lengths.

In this and selected other examples, composite material manager **212** can predict a set of properties **228** that portions **226** of unconsolidated composite material **204** will have when manufacturing of unconsolidated composite material **204** is complete. In other words, this prediction is made while portions **226** of unconsolidated composite material **204** is still being manufactured. Different portions in portions **226** can be in different states or stages of manufacturing in composite material manufacturing system **206.**

The set of properties **228** can take a number of different forms. For example, the set of properties **228** comprises at least one of a prepreg fiber areal weight, prepreg resin content, prepreg thickness, prepreg infiltration level, a prepreg tack level, resin film areal weight, resin film thickness, release liner areal weight, release liner thickness, or a laminate structural property

Composite material manager **212** identifies quality level **230** for the number of portions **226** of unconsolidated composite material **204** based on the set of predicted properties **224** for the number of portions **226** of unconsolidated composite material **204.** This identification of quality level **230** is predicted quality level **232.** In other words, quality level **230** is determined using predicted properties **224.** Actual testing of the number of portions **226** can be performed to determine actual properties for determining the actual quality level.

In this and selected other examples, the determination of the set of predicted properties **224** can be made by composite material manager **212** using a set of models **233.** The set of models **233** can include at least one of a set of machine learning models **234,** a set of physics models **235,** a set of hybrid models **237,** or other suitable type of models. In this and selected other examples, any combination of models **233** can used to determine predict properties **228.**

When a set of machine learning models **234** are used, the set of machine learning models **234** can be trained to predict properties **228** for a particular type of unconsolidated composite material **204** using sensor data **218.** A machine learning model is a type of artificial intelligence model and is a data driven model that can learn without being explicitly programmed. A machine learning model can learn based on training data input into the machine learning model. The machine learning model can learn using various types of machine learning algorithms. The machine learning algorithms include at least one of a supervised learning, and unsupervised learning, a feature learning, a sparse dictionary learning, an anomaly detection, a reinforcement learning, a recommendation learning, or other types of learning algorithms.

Examples of machine learning models include an artificial neural network, a decision tree, a support vector machine, a random forest machine learning model, a regression machine learning model, a classification machine learning model, a Bayesian network, and other types of models. These machine learning models can be trained using data and process additional data to provide a desired output.

For example, machine learning model **236** can be trained to determine predicted properties **224** for unconsolidated composite material **204** such as prepreg using training dataset **240.** Training dataset **240** can be created using historical sensor data **242** received from composite material manufacturing system **206** for manufacturing unconsolidated composite material **204** in the form of a prepreg. Training dataset **240** can include labels in the form of historical properties **244.** Historical properties **244** can be determined from measurements made of unconsolidated composite material **204** at different locations in composite material manufacturing system **206.** In other words, historical properties **244** can include properties **228** of unconsolidated composite material **204** when manufacturing of unconsolidated composite material **204** has been completed.

Additionally, training dataset **240** can also include historical upstream information **245.** Historical upstream information **245** can be associated with historical sensor data **242** generated when manufacturing an unconsolidated composite material in which the manufacturing process includes historical upstream information. Historical upstream information **245** can include information about components or settings used in manufacturing unconsolidated composite materials. For example, historical upstream information **245** can be selected from at least one of reinforcement information, fiber information, reinforcement sizing information, resin information, release liner information, resin film information, or other suitable information that may be measured to generate historical sensor data **242.**

Properties **228** of interest can take number of different forms depending on the type of unconsolidated composite material **204.** For example, when unconsolidated composite material **204** is a prepreg, properties **228** can include at least one of thickness, fiber areal weight (FAW), resin weight content (RC), prepreg areal weight (PAW), water pickup, tack, pucker, or other properties of interest for unconsolidated composite material **204** in the form prepreg.

With the use of machine learning model **236** to determine predicted properties **224,** composite material manager **212** can predict these properties prior to completing manufacturing of unconsolidated composite material **204.** In other words, predicted properties **224** for unconsolidated composite material **204** in a completed or manufactured state can be made during different locations in different times during the manufacturing of unconsolidated composite material **204.**

In some other examples, a set of physics models **235** can be used to determine predicted properties **224** for unconsolidated composite material **204.** In this and selected other examples, physics model **239** in the set of physics models **235** is a mathematical model that contains equations that can describe the effects of actions performed on materials to manufacture unconsolidated composite material based on at least mathematical equations. These actions can be, for example, the in response to parameters such heat, tension, pressure, force, or other parameters in sensor data **218.**

The set of physics models **235** can take a number of different forms. For example, the set of physics models can be selected from at least one of a machine dynamics thermal and mechanical model, a resin infiltration model, a Darcy infiltration model including viscosity, a resin flow model, a cure kinetics model, a finite element analysis (FEA) model of infiltration, a finite element analysis (FEA) model of layup and other suitable types of models physics based models.

The set of hybrid models **237** are hybrid physics based and data driven models that use both physics component and machine learning components. In this and selected other examples, hybrid models **237** combines physics (real time software) or measured data along with learned/predicted data into a single model such that the distribution/noise for the sensors embedded in the predictions along with other items being evaluated.

Statistical models **243** can also be present in models **233.** Statistical models **243** can be selected from at least one of a Kalman filter, an extended Kalman filter, an unscented Kalman filter, or other suitable statistical models. For example, Kalman filters can be used with noisy data to estimate the property of a system that is being measured to increase the accuracy of the estimate of the property being measured.

With the prediction of when determining predicted properties **224** for a number of portions **226** of unconsolidated composite material **204,** composite material manager **212,** can determine quality level **230** for the number of portions **226** of unconsolidated composite material **204** using the set of predicted properties **224** determined for the number of portions **226** of unconsolidated composite material **204.** In this and selected other examples, different portions in portions **226** can have a different quality level from other portions.

With the identification of quality level **230,** composite material manager **212** can perform a number of actions **241.** Depending on quality level **230** identified for each portion in the number of portions **226,** a different action in actions **241** can be performed for that portion.

Further, other information can be used in addition to sensor data **218** for determining predicted properties **224** for the number of portions of unconsolidated composite material **204.** For example, upstream information **246** can be used with sensor data **218** by composite material manager **212** to determine predicted properties **224** for the number of portions **226.** Upstream information **246** can be selected from at least one of reinforcement information, fiber information, reinforcement sizing information, resin information, release liner information, resin film information, or other suitable information that can be used with sensor data **218** to determine predicted properties **224** for number of portions **226** of unconsolidated composite material **204.**

Models **233** can operate as digital twin **203** and can be used by composite material manager **212** to determine predicted properties **224** for a number of portions **226** of unconsolidated composite material **204.** As a result, the placement of sensors **222** in sensor system **220** in locations **209** for composite material manufacturing system **206** can provide sensor data **218** used by models **233** for digital twin **203** to predict a number of properties **228** for a number of portions **226** of unconsolidated composite material **204.** Using models **233** as digital twin **203** enables real time inspection and certification of a number of portions **226** of unconsolidated composite material **204** that can identify properties **228** of the number of portions **226** of unconsolidated composite material **204** without having to sample the number of portions **226** of unconsolidated composite material **204.** If actual testing is desired, the identification of portions **226** of unconsolidated composite material **204** can be tested to verify the predictions if needed.

Further, any combination of different types of models **233** can be used to provide increased accuracy in predicted properties **224.** For example, a first set of initial predicted properties **251** can be determined for a portion of unconsolidated composite material **204** from the machine learning model **236** generated using sensor data **218.** A second set of initial predicted properties **251** can be determined for the same portion of unconsolidated composite material **204** from physics model **239** generated using sensor data **218.** The set of predicted properties **224** for the portion of unconsolidated composite material **204** can be determined from a fusion of the first set of initial predicted properties and the second set of initial predicted properties.

With the determination of the set of predicted properties composite material manager **212** can determine quality level **230** for a number of portions **226** of unconsolidated composite material **204** using the set of predicted properties **224.** Quality level **230** is predicted quality level **232** that the number of portions **226** of unconsolidated composite material **204** is predicted or expected to have when manufacturing of the number of portions **226** has been completed and the number of portions **226** of unconsolidated composite material **204** forms completed product **227.**

Composite material manager **212** can perform a number of actions **241** based on quality level **230** predicted for the number of portions **226** of unconsolidated composite material **204.** In this and selected other examples, quality level **230** is determined using the number of predicted properties **224** for the number of portions **226** of unconsolidated composite material **204.** For example, the number of actions **241** can include corrective action **291.** Composite material manager **212** can perform corrective action **291** based on quality level **230** for a number of portions **226** of unconsolidated composite material **204.**

In some examples including this depicted example, corrective action **291** can be performed prior to completing manufacturing of the number of portions **226** of unconsolidated composite material **204** to form completed product **227.** In some examples including the illustrative example, manufacturing of the number of portions **226** of unconsolidated composite material **204** can be completed prior to or after other portions in portions **226** of unconsolidated composite material **204.** In other words, number of portions **226** of unconsolidated composite material **204** can be completed product **227** while other portions are not yet completed product **227** and are still being processed within composite material manufacturing system **208.**

As a result, increased number of portions **226** of unconsolidated composite material **204** can be used for manufacturing composite part **205** when corrective action **291** is performed. For example, at least one of discarding, reworking, or recycling the number of portions **226** can be avoided by performing corrective action **291** prior to completing manufacturing a number of portions **226** of unconsolidated composite material **204.**

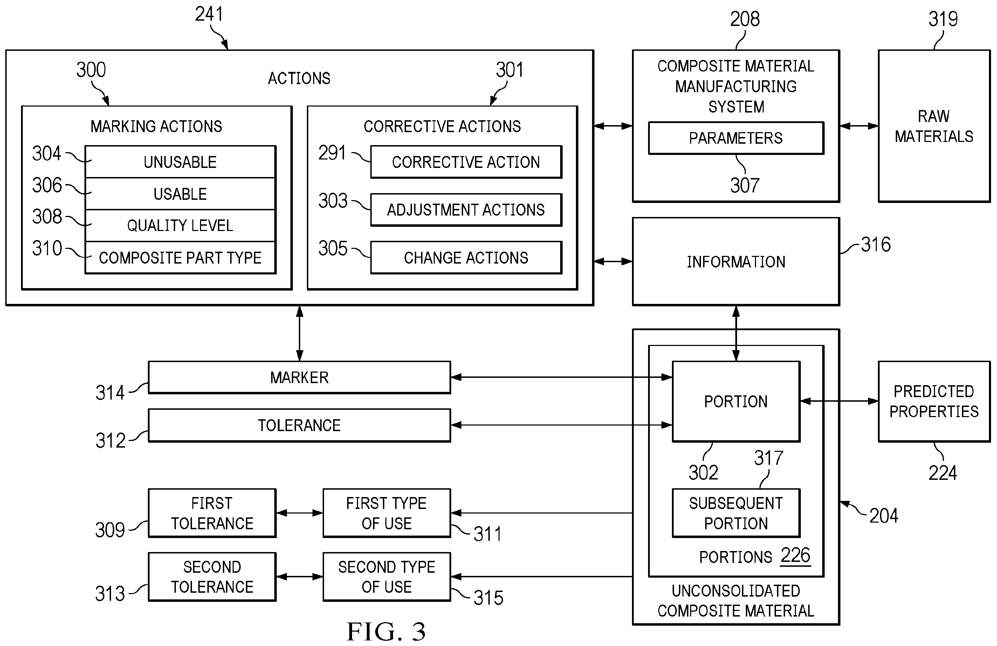

With reference next to **Figure 3****,** an illustration of a block diagram of actions that can be taken using predicted properties for portions of an unconsolidated composite material is depicted in accordance with an illustrative example. In some examples including the illustrative examples, the same reference numeral may be used in more than one figure. This reuse of a reference numeral in different figures represents the same element in the different figures.

As depicted, actions **241** can take a number of different forms. For example, actions **241** can be performed for the number of portions **226** of unconsolidated composite material **204** as manufacturing of the number of portions **226** are completed.

Actions **241** can include marking actions **300.** As depicted marking actions **300** can include marking portion **302** of unconsolidated composite material **204** in the number of portions **226** as unusable **304,** usable **306,** quality level **308,** and composite part type **310.**

In this and selected other examples, unusable **304** is an action in which composite material manager **212** marks portion **302** of unconsolidated composite material **204** in the number of portions **226** of unconsolidated composite material **204** as unusable based on quality level **230** for portion **302** being out of tolerance **312.** In this and selected other examples, tolerance **312** can be a range or a threshold for one or more properties **228** of portion **302** that are considered acceptable or tolerable. The property or properties of interest are in tolerance **312** can be used to determine whether quality level **308** for portion **302** is acceptable.

As depicted, usable **306** is an action in which composite material manager **212** marks portion **302** of unconsolidated composite material **204** in the number of portions **226** of unconsolidated composite material **204** as usable based on quality level **230** for portion **302** being in tolerance **312.** Quality level **308** is an action in which composite material manager **212** marks portion **302** of unconsolidated composite material **204** in the number of portions **226** of unconsolidated composite material **204** with the quality level identified for portion **302.**

As another example, composite part type is an action in which composite material manager **212** marks portion **302** of unconsolidated composite material **204** in the number of portions **226** of unconsolidated composite material **204** with an identification of the type of composite part that portion **302** can be used to manufacture. For example, the quality level may be such that the portion **302** is unsuitable for use for the intended type of composite part. However, the quality level may be such that portion **302** can be used to manufacture another type of composite part. As a result, portion **302** can still be used rather than discarded or recycled.

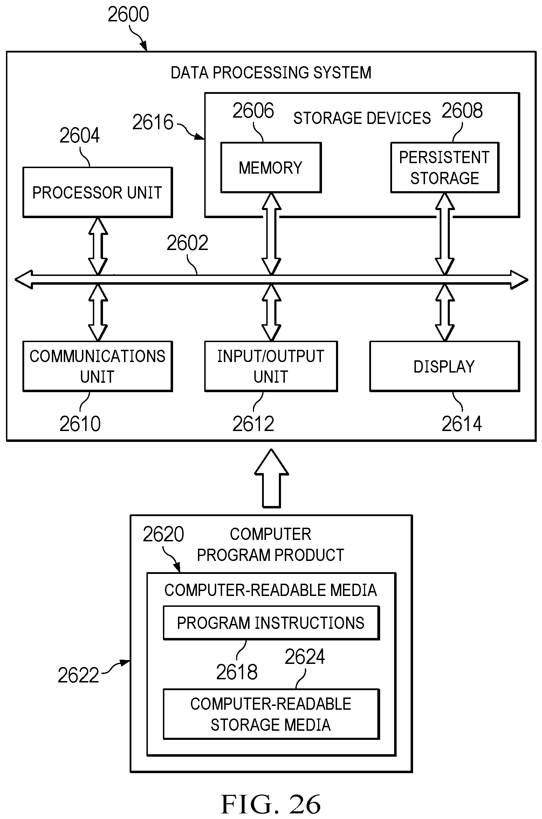

The identification of these actions can be marked on portion **302** using marker **314.** Marker **314** can be an identifier such as a radiofrequency (RFID) identifier, a microdot, a set of quantum dots, an ink mark, a barcode, a quick response (QR) code, or some other suitable type of marker. Marker **314** can identify the action. Marking actions **300** can also include recording information **316** about portion **302** using marker **314.** For example, the information can include the length of portion **302,** location of portion **302** in unconsolidated composite material **204,** quality level, predicted properties **224,** shelf life, or other information about portion **302.** Information **316** can be determined from predicted properties **224** for portion **302.** Markers **314** and marking actions **300** also include recording the position on the roll or recording information **316** about portion **302** in a memory **2606** as shown in **Figure 26** for later correlation of quality of portion **302** to a position on a roll. One example of this use is to print out position information that designates the quality on the roll at specific positions on the roll. In other words, marking can include at least one of indicating or recording information in a record or log about the quality in addition to or in place of actually marking portion **302** itself. As a result, the result of marking can also be a printout or a thumb drive that accompanies each roll with the "marking".

In some examples including this illustrative example, actions **241** can also include corrective actions **301.** Corrective actions **301** are actions **241** that can be performed on a number of portions **226** including portion **302.** In some examples including this illustrative example, corrective action **291** in the set of corrective actions **301** can be performed by composite material manager **212** in response to the set of predicted properties **224** for the number of portions **226** being out of tolerance **312** specified for unconsolidated composite material **204.**

These corrective actions can be performed in an effort to change the set of predicted properties **224** for the number of portions **226** such that the set of predicted properties **224** are within a tolerance **312** for the number of portions **226** when manufacturing of the number of portions **226** of unconsolidated composite material **204** as completed to form completed product **227.**

As depicted, corrective actions **301** can take a number of different forms. For example, corrective actions **301** can include at least one of adjustment actions **303,** change actions **305,** or some other suitable corrective actions.

For example, when corrective action **291** in corrective actions **301** takes the form of adjustment actions **303,** composite material manager **212** can adjust a set of parameters **307** in the composite material manufacturing system **208** to change the set of predicted properties for the number of portions of the unconsolidated composite material in response to the set of predicted properties **224** for number of portions **226** being out of tolerance **312** specified for unconsolidated composite material **204.**

In some examples including this depicted example, set of parameters for composite material manufacturing system **208** can take a number of different forms. For example, the set of parameters **307** can be selected from at least one of a speed, a torque, a voltage, a current, a gap, a nip gap, a force, a tension, a thickness at a location, a pressure or other suitable parameters that can be set in composite material manufacturing system **208.**

For example, corrective action **291** can be jesting a set of parameters **307** in composite material manufacturing system **208.** Adjustment of the set of parameters **307** can be such that set of predicted properties **224** for the number of portions **226** of unconsolidated composite material **204** is within tolerance **312** for the unconsolidated composite material **204.**

In yet other examples, adjustments may not make the set of predicted properties **224** to fall within tolerance **312** for the number of portions **226** of unconsolidated composite material **204** to meet tolerance **312** for the specified use for unconsolidated composite material **204.** In some examples including this depicted example, the set of predicted properties **224** for the number of portions **226** of unconsolidated composite material **204** is outside of first tolerance **309** for a first type of use **311** of unconsolidated composite material **204.**

In some examples including this example, performing corrective action **291** based on quality level **230** for the number of portions **226** of unconsolidated composite material **204** comprises adjusting a set of parameters **307** in composite material manufacturing system **208** such that the set of predicted properties **224** for the number of portions **226** of unconsolidated composite material **204** is within second tolerance **313** for second type of use **315** of unconsolidated composite material **204.**

For example, first type of use **311** can be for using unconsolidated composite material **204** in a skin panel. Second type of use **315** can be for using unconsolidated composite material **204** in a monument within an aircraft.

This type of corrective action **291** can also be performed to create some portions of unconsolidated composite material **204** for second type of use **315** instead of first type of use **311.** In other words, this action can also be performed even when a set of predicted properties **224** is within tolerance **312.** As result, when the need arises for unconsolidated composite materials **204** for second type of use **315,** some of unconsolidated composite material **204** is manufactured for first type of use **311** can be diverted for second type of use **315.**

In other illustrative examples, corrective action **291** can comprise adjusting a set of parameters **307** in composite material manufacturing system **208** such that the set of predicted properties **224** for subsequent portion **317** to the number of portions **226** of unconsolidated composite material **204** is within second tolerance **313** for unconsolidated composite material **204** in response to the set of predicted properties **224** for the number of portions **226** being out of tolerance **312** specified for unconsolidated composite material **204.** This type of corrective action can be performed when changing parameters **307** will not cause the number of portions **226** of unconsolidated composite material **204** to have set of predicted properties **224** that fall within tolerance **312.** However, this corrective action can be used to cause later portions of unconsolidated composite materials **204** to have a set of predicted properties **224** that fall within tolerance **312.** As result, a marking action is still performed for the number of portions **226** in this example.

In other illustrative examples, corrective action **291** can be a change action in change actions **305.** For example, corrective action **291** can change a number of raw materials **319** used to form unconsolidated composite material **204.** The change in the number of raw materials **319** is such that the set of predicted properties **224** for subsequent portion can be the number of portions **226** of unconsolidated composite material **204** that is within tolerance **312** for unconsolidated composite material **204** in response to the set of predicted properties **224** for the number of portions **226** being out of tolerance **312** specified for the unconsolidated composite material **204.**

With this and selected other examples, a raw material in raw materials **319** can be any material that is used by composite material manufacturing system 208 to manufacture unconsolidated composite material **204.** For example, the number of raw materials **319** can be selected from at least one of a resin, a paper, a film, a fabric, a tow, a spread tow fabric, a fiber type, a tow size, a fiber size, or other material used to manufacture unconsolidated composite material **204.**

In some illustrative examples, multiple corrective actions can be taken. For example, both in adjustment action and a change action can be performed.

In some illustrative examples, one or more solutions are present that overcome a problem with an inability to determine properties of unconsolidated composite materials as quickly as desired. One or more illustrative examples can enable predicting properties of different portions of unconsolidated composite materials before manufacturing of those portions have been completed. With this type of prediction, action such as marking different portions or performing other actions may be made in a more quickly than testing samples after manufacturing of the unconsolidated composite materials has been completed.

Computer system **210** can be configured to perform at least one of the steps, operations, or actions described in the different illustrative examples using software, hardware, firmware or a combination thereof. As a result, computer system **210** operates as a special purpose computer system in which composite material manager **212** in computer system **210** enables predicting properties of an unconsolidated composite material during manufacturing of the unconsolidated composite material before the manufacturing of unconsolidated composite material has completed. The use of real time data about the unconsolidated composite material during manufacturing of the unconsolidated composite material enables determining predicted properties for the unconsolidated composite material during manufacturing of the unconsolidated composite material. Further, using a machine learning model enables determining predicted properties of the unconsolidated composite material before manufacturing of the unconsolidated composite material has been completed. In other words, determination of the predicted properties does not require sampling and testing the unconsolidated composite material after manufacturing of unconsolidated composite material has been completed. Further this determination cannot be performed human operator quickly enough to obtain the prediction of the properties for the unconsolidated composite material before manufacturing of unconsolidated composite material has been completed.

In particular, composite material manager **212** transforms computer system **210** into a special purpose computer system as compared to currently available general computer systems that do not have composite material manager **212.**

In some examples including the illustrative example, the use of composite material manager **212** in computer system **210** integrates processes into a practical application for manufacturing an unconsolidated composite material that increases the performance of computer system **210** in predicting properties **228** for portions **226** of unconsolidated composite material **204.** In other words, composite material manager **212** in computer system **210** is directed to a practical application of processes integrated into composite material manager **212** in computer system **210** that generate predictions of properties **228** for unconsolidated composite material **204** in real time such that actions **241** can be taken more quickly with respect to unconsolidated composite material **204.** This process using composite material manager **212** to generate predicted properties **224** using models **233** from sensor data **218** received in real time can be performed in real time to obtain predicted properties **224** before manufacturing unconsolidated composite material **204** is complete, which is not possible with human operators analyzing sensor data **218** by hand.

In this and some other examples, composite material manager **212** in computer system **210** enables predicting properties for unconsolidated composite material such as prepreg or resin coated film, before completing manufacturing of the unconsolidated composite material. As a result, actions can be performed on the portions as the manufacturing of those portions are being completed.

For example, the different portions of the unconsolidated composite material can be marked as different portions are completed to indicate suitability or quality of those portions of the unconsolidated composite material. Composite material manager **212** provides a practical application of managing unconsolidated composite materials as the unconsolidated composite materials are manufactured using sensor data **218** obtained from different locations in locations **209** for composite material manufacturing system **206.** Predicted properties **224** can be used to mark portions **226** of unconsolidated composite material **204.** The marking can be performed based on predicted quality level **232** using predicted properties **224.**

This type of management is in contrast to current techniques that may take a sample of a prepreg material in a role to determine the quality of the entire roll. Composite material manager **212** provides an improvement in which this determination can be made for different portions of the prepreg roll and can be performed as the prepreg roll is being manufactured. As result, the reduction in amount of time needed to manufacture unconsolidated composite materials is realized using composite material manager **212** to predict quality level portions of unconsolidated composite material.

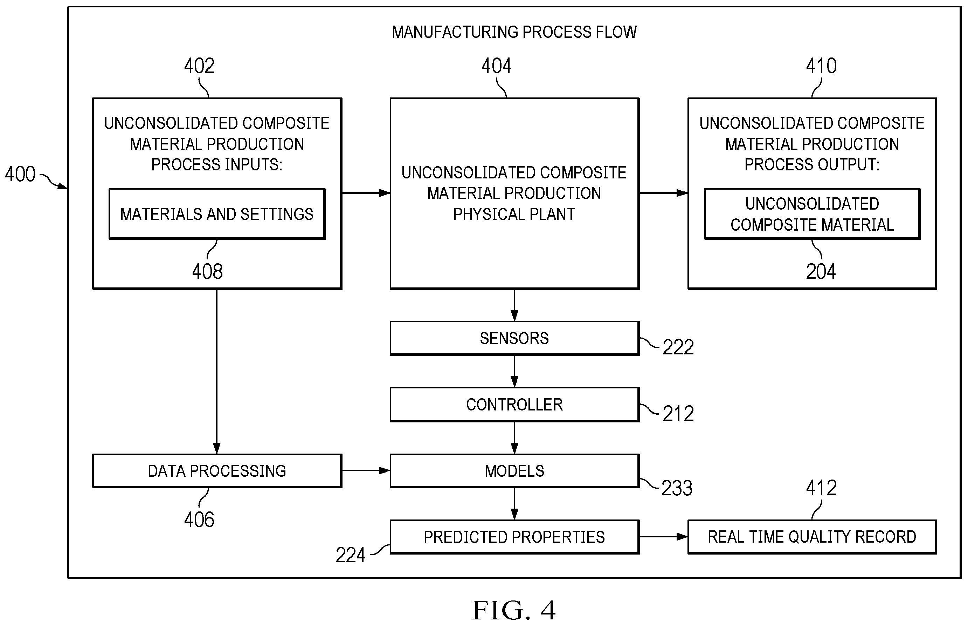

With reference next to **Figure 4****,** an illustration of a manufacturing process flow for manufacturing unconsolidated composite materials is depicted in accordance with an illustrative example. In this and some other example, manufacturing process flow **400** begins with unconsolidated composite material production process inputs **402** being made to unconsolidated composite material production physical plant **404** and to data preprocessing **406.** These inputs in unconsolidated composite material production process inputs **402** include materials and settings **408.** In this and some other example, materials and settings **408** are information that identify materials to be used in manufacturing unconsolidated composite material **204.** When unconsolidated composite material **204** is prepreg, the materials can be for example, fiber type, fiber size, resin film specifications, reinforcement information, fiber information, reinforcement sizing information, resin information, release liner information, resin film information, or other suitable information about materials used to manufacture the prepreg.

The settings can be information such as temperature, pressure, tension, and other information that can be used for settings or to a configure a composite material manufacturing system in unconsolidated composite material production physical plant **404** to manufacture the prepreg. Materials and settings **408** can be part of upstream information **246.**

With materials and settings **408** as an input, unconsolidated composite material production physical plant **404** can perform manufacturing operations to generate unconsolidated composite material production process output **410** in the form of unconsolidated composite material **204.** These operations can be performed using equipment such as composite material manufacturing systems in the plant. In this and some other examples, portions of unconsolidated composite material **204** can be output as unconsolidated composite material production process output **410** as manufacturing of portions of unconsolidated composite material **204** are completed.

Data preprocessing **406** can be a component located in computer system **210** that processes unconsolidated composite material production process inputs **402** for use by a number of models **233** to determine predicted properties **224.** For example, data preprocessing **406** can process materials and settings **408** to form upstream information **246** that is input into the number of models **233.**

Sensors **222** monitors the manufacturing of unconsolidated composite material **204** in real time as unconsolidated composite material **204** is being manufactured and sends sensor data to composite material manager **212** in real time as unconsolidated composite material **204** is being manufactured. Composite material manager **212** acquires sensor data from sensors **222** and sends that sensor data into a number of models **233.**

In this and some other examples, the sensor data can be sent to a single model in models **233** or multiple models in models **233.** When a number of models **233** is a single model, the model can be a machine learning model. When the number of models **233** is multiple those models can be, for example, two machine learning models, a machine learning model and a physics model, or some combination of models. When more than one model in models **233** is used, the outputs of those models can be compared to identify a best prediction for predicted properties **224** or combined or fused to form predicted properties **224.**

Models **233** generate predicted properties **224.** This prediction is made in real time prior to the completion of manufacturing of unconsolidated composite material **204.** For example, predicted properties **224** can be for a portion of unconsolidated composite material **204** for which manufacturing has not been completed. In other words, predicted properties **224** can be determined for the portion of unconsolidated composite material **204** prior to that portion being output as a completed product. The completed product can be used to manufacture composite part **205.**

In this and some other examples, predicted properties **224** can be used to perform an action in actions **241** in the form of real time quality record **412.** This record can be used to track portions of unconsolidated composite material **204** for performing additional manufacturing operations to manufacture other unconsolidated composite materials or composite parts.

The illustration of composite manufacturing environment **200 in** **Figures 2-4** is not meant to imply physical or architectural limitations to the manner in which an illustrative example can be implemented. Other components in addition to or in place of the ones illustrated may be used. Some components may be unnecessary. Also, the blocks are presented to illustrate some functional components. One or more of these blocks may be combined, divided, or combined and divided into different blocks when implemented in an illustrative example.

For example, composite material manager **212** can be used to predict properties of one or more unconsolidated composite materials in addition to unconsolidated composite material **204.** These other unconsolidated composite materials can be manufactured using other composite material manufacturing systems in addition to composite material manufacturing system **208.** Further, the additional unconsolidated composite materials manufactured using composite material manufacturing systems can be different types of unconsolidated composite materials manufactured using different types of composite material manufacturing systems.

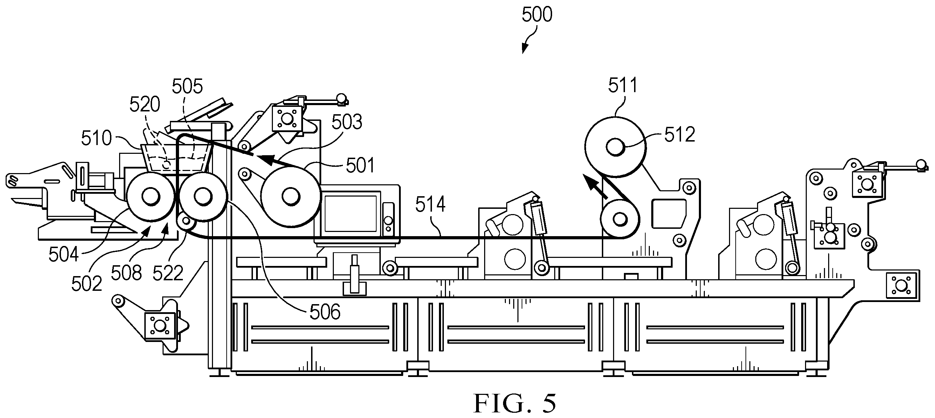

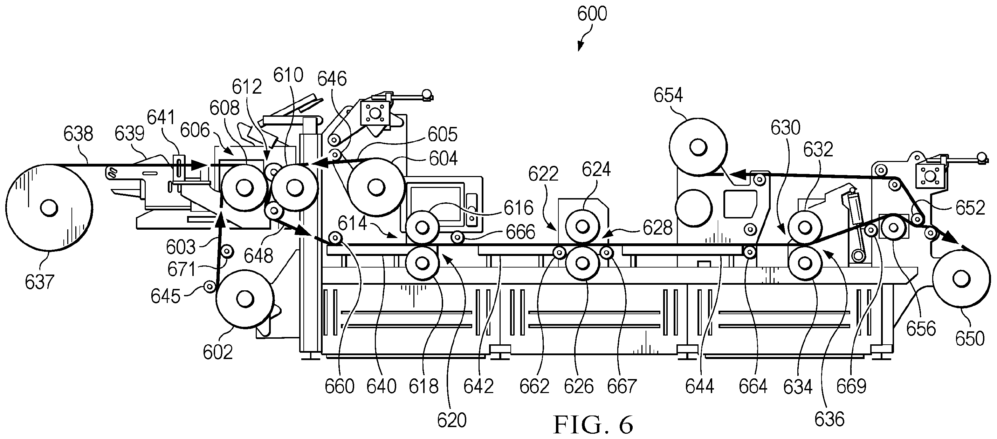

**Figure 5** and **Figure 6** are examples of composite material manufacturing systems that can be used to implement composite material manufacturing system **206** in **Figure 2****.** A sensor system can be added to these composite material manufacturing systems or existing sensor systems can be augmented to include one or more sensors in configurations that can generate the sensor data needed to predict properties for unconsolidated composite materials as the unconsolidated composite materials are being manufactured by these composite material manufacturing systems.

With reference now to **Figure 5****,** an illustration of a composite material manufacturing system configured for performing filming process is depicted in accordance with an illustrative example. Composite material manufacturing system **500** shown in block form in **Figure 2****.**

As depicted, composite material manufacturing system **500** is configured to perform a filming process in which resin is coated onto film paper.

In this and some other examples, unfilmed paper roll **501** feeds paper **503** into nip **502.** A nip comprises a pair of rollers with a gap set between the rollers. As depicted nip **502** comprises roller **504** and roller **506** with gap **508** between roller **504** and roller **506.** The gap can be fixed or change to provide constant pressure.

Resin **505** is heated and located in resin dams **510** that sit on roller **504** and roller **506.** Resin dams **510** feeds resin **505** into gap **508** while paper **503** is pulled through gap **508** and onto filmed paper roll **511** by paper rewinder **512.** Paper **503** becomes filmed paper **514** as resin **505** from resin dams **510** coats paper **503** at gap **508.** Filmed paper **514** is collected on filmed paper roll **511.**

In this and some other example, the properties of filmed paper **514** can be predicted using sensor data generated by sensor system for composite material manufacturing system **500.** For example, the sensor system can measure tension on paper **503,** the speed at which paper **503** moves, a gap width of gap **508,** resin viscosity of resin **505** in resin dams **510,** basis/areal weight, and other parameters. The gap width of gap **508** and resin viscosity of resin in resin dams **510** can affect how much resin is coated onto paper **503** to form filmed paper **514.** Resin viscosity is the thickness of the liquid resin and is typically measured in centipoise (cP).

Resin viscosity of resin **505** in resin dams **510** can be measured using a sensor **520** in resin dams **510.** Sensor **520** can be a viscometer. The measurement of resin viscosity with the gap width of gap **508** can be used to predict the thickness of resin **505** on filmed paper **514.** As another example, resin viscosity can be determined using dielectric measurements. For example, sensor **520** can apply an oscillatory voltage to resin **505** in resin dams **510** and measure the current. This type of measurement can also be made by sensor **522** located just after gap **508** in which sensor **522** applies an oscillatory voltage and measures current. This measurement can be used to predict the amount of resin that will be present on filmed paper **514** that is collected on filmed paper roll **511.**

When creating a training dataset, the sensor data can be labeled with actual measurements of resin on filmed paper **514.** For example, the paper from each roll can be premeasured at the beginning at unfilmed paper roll **501** and at the end at filmed paper roll **511.** The weights of the two rolls can be subtracted to obtain the weight difference to determine the amount of resin. This measurement provides the overall resin on all of filmed paper **514** and not portions of filmed paper **514.** Samples for portions can be taken in which a fixed area paper is cut out and weighed for different portions. The weight of the paper is known and that weight can be subtracted to measure the weight of the sample to determine the weight of the resin. The difference between the paper and resin can be identified to obtain the amount of resin on the sample.

Turning to **Figure 6****,** an illustration of a composite material manufacturing system configured for performing a prepreg production process is depicted in accordance with an illustrative example. In this and some other examples, composite material manufacturing system **600** is another example of an implementation of composite material manufacturing system **208** shown in block form in an in **Figure 2****.** Composite material manufacturing system **600** is configured to manufacture and unconsolidated composite material in the form of prepreg. The prepreg can be manufactured using a hot melt prepregging process or a solvent-based prepreg process.

As depicted, composite material manufacturing system **600** has two filmed rolls, filmed roll 1 **602** and filmed roll 2 **604.** The filmed paper on these rolls is paper with resin. For example, filmed roll 1 **602** is a source of filmed paper **603** and filmed roll 2 **604** is a source of filmed paper **605.** These filmed rolls can be manufactured using composite material manufacturing system **500 in** **Figure 5****.**

In this example, composite material manufacturing system **600** has four nips. Nip1 **606** has roller **608** and roller **610** with nip1 gap **612** between these two rollers. Nip2 **614** has roller **616** and roller **618** with nip2 gap **620.** Nip3 **622** has roller **624** and roller **626** with nip3 gap **628.** Nip4 **630** has roller **632** and roller **634** with nip1 gap **636.** In this and some other examples, these nips operate are squeeze points where pressure can be applied to materials traveling through the nips.

As depicted, hotplate **640** and hotplate **642** are present in composite material manufacturing system **600.** This system also has cold plate **644.**

In this and some other examples, nip1 **606,** nip2 **614,** nip3 **622,** nip4 **630,** hotplate **640,** and hotplate **642** are kept at set temperatures. The temperatures of each of these components can be set separately from other components. In other words, the temperature of nip1 **606** can be set to a different temperature from nip2 **614.**

In this and some other examples, fiber creel **637** is a source of fibers **638.** These fibers are feed through comb **639** and onto spreader bars **641** before being moved into nip1 **606.**

As depicted, filmed paper **603** and filmed paper **605** both move into nip1 **606** from filmed roll 1 **602** and filmed roll 2 **604,** respectively. Brake **645** on filmed roll 1 **602** maintains tension for filmed paper **603** and brake **646** on filmed roll 2 **604** maintain tension for filmed paper **605** such that prepreg **648** does not go slack.

The side of filmed paper **603** and the side of filmed paper **605** having resin faces fibers **638.** In this and some other examples, the temperature and pressure are applied by nip1 **606** to cause the resin to infiltrate from filmed paper **603** and filmed paper **605** into fibers **638** at nip1 **606.**

For example, the width of nip1 gap **612** in nip1 **606** is selected to apply pressure to fibers **638** sandwiched between the resin on filmed paper **603** and filmed paper **605.** Further, roller **608** and roller **610** for nip1 **606** has a temperature selected to heat fibers **638** and the resin on filmed paper **603** and filmed paper **605.** The at least one of the heat or pressure causes the resin to infiltrate fibers **638** to form prepreg **648** when fibers **638,** filmed paper **603,** and filmed paper **605** exit nip1 **606.**

The other nips, nip2 **614,** nip3 **622,** nip4 **630,** can have nip gaps and roller temperatures set to provide the desired amount of pressure and heating as prepreg **648** travels in composite material manufacturing system **600** to prepreg roll **650.** The nip gap and roller temperatures can change the viscosity of the resin and in turn change the speed of the infiltration process. Hotplate **640** is located between nip1 **606** and nip2 **614,** and hotplate **642** is located between nip2 **614** and nip3 **622.** These hotplate heat prepreg **648** as prepreg **648** travels between nips. Cold plate **644** is located after nip3 **622** and before nip4 **630.** This cold plate can lower the temperature of prepreg **648.** Cold plate **644** can reduce the temperature which increases the viscosity of the resin to reduce or stop the infiltration process of resin into the fibers.

The width of prepreg **648** can be set by slitters **656** before filmed paper **605** is removed. At roller **652,** filmed paper **605** is removed and wound onto paper roll **654.** Prepreg **648** is now comprised of fibers **638** infused with resin on filmed paper **603** and wound onto prepreg roll **650.**

In this and some other examples, sensors can be located at different locations between points in composite material manufacturing system **600.** In this and some other examples, these points can be processing points where actions can be performed to manufacture prepreg **648.** These processing points can be, for example, nip1 **606,** nip2 **614,** nip3 **622,** nip4 **630.** Other processing points can be hotplate **640,** hotplate **642,** cold plate **644** where heat or cold is applied to prepreg **648** to heat or cool prepreg **648.**

Processing points can change properties of prepreg **648** as prepreg **648** is manufactured prepreg **648** in its final form at prepreg roll **650.** Sensors can be located between these points and can be used to predict the properties of prepreg **648** as completed from manufacturing prepreg **648** at prepreg roll **650.** In other words, with sensor data from sensors between these processing points, predicted properties can be determined for prepreg **648** at prepreg roll **650** from other locations in composite material manufacturing system **600** prior to prepreg roll **650.**

In some examples, sensor location **660** is located between nip1 **606** and nip2 **614.** In some other examples, sensor location **662** is located between nip2 **614** and nip3 **622,** and sensor location **664** is located between nip3 **622** and hotplate **640.** In yet other examples, sensor location **666** is located between nip2 **614** and hotplate **642. In** yet other examples, sensor location **667** is located between nip3 **622** and cold plate **644.**

In this and some other examples, another sensor location, sensor location **671** can be located between filmed roll 1 **602** and nip1 **606.** In other examples, sensor location **669** is located between nip4 **630** and prepreg roll **650.** The sensor location is an example of a sensor location that can be used even though the location is not between two processing points. Sensor data about of filmed paper **603** can be used in predicting properties of prepreg **648** at prepreg roll **650.**

In this and some other examples, composite material manufacturing system **600** is a hot-melt film prepreg system. This illustrative prepreg system is not meant to limit the types of prepreg systems that can be use. Other examples can encompass those other types of prepreg systems that utilize bath, slurry, spray, or other mechanism to apply resin to the carrier fibers.

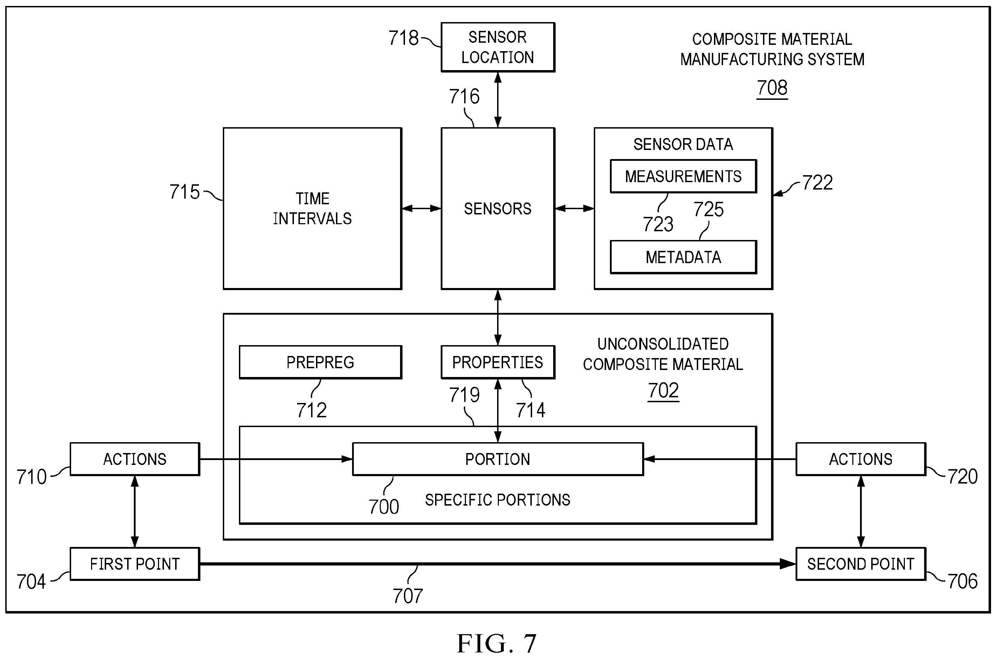

Reference next to **Figure 7****,** an illustration of a block diagram of a sensor location in a composite material manufacturing system is depicted in accordance with an illustrative example. In this and some other examples, portion **700** of unconsolidated composite material **702** travels from first point **704** to second point **706** in the direction of arrow **707** in composite material manufacturing system **708.**

In this and some other examples, a set of actions **710** can be performed on portion **700** of unconsolidated composite material **702** at first point **704** as portion **700** of unconsolidated composite material **702** passes first point **704.** The set of actions **710** can be one or more actions performed in manufacturing unconsolidated composite material **702.** In this and some other examples, the set of actions **710** can be selected from at least one of applying heat, applying pressure, applying a tension, applying a force, applying cold, changing a viscosity, or other suitable action.

For example, when unconsolidated composite material **702** is prepreg **712** and first point **704** is the location component such as a nip having rollers, the set of actions **710** can be apply pressure and heat to portion **700.** The set of actions **710** result in a set of properties **714** being present after first point **704.** The set of properties **714** can change from what was present prior to the set of actions **710** being performed on portion **700** at first point **704.**

The set of properties **714** for portion **700** can be different from the properties present prior to the set of actions being performed on portion **700** at first point **704.** In other words, the set of actions performed at first point **704** can change the set of properties **714** for portion **700** of unconsolidated composite material **702.**

The set of properties **714** present after the performing the set of actions **710** on portion **700** of unconsolidated composite material **702** can be detected using a set of sensors **716** at sensor locations **718.** The set of sensors **716** can generate sensor data **722.** Sensor data **722** can include measurements **723** and metadata **725.** When metadata **725** is present in sensor data **722.** Metadata **725** can include, for example, at least one of a timestamp, a sensor location, a sensor identifier, or other suitable information about measurements **723.**

When unconsolidated composite material **702** is prepreg **712,** the measurements **723** in sensor data **722** can be used to determine the amount of resin present in an area such as portion **700.** For example, measurements **723** in sensor data **722** can be aerial weight. The current amount of resin present can be used to predict the amount of resin that will be present before the manufacturing process for portion **700** of unconsolidated composite material **702** is complete.

This sensor data can be used to predict properties **714** that portion **700** of unconsolidated composite material **702** will have when the manufacturing of portion **700** of unconsolidated composite material **702** is completed. The set of sensors **716** can be one or more of sensors **222** in sensor system **220** for composite material manufacturing system **206.**

In this and some other examples, sensor location **718** is located after first point **704** and prior to second point **706.** Sensor location **718** can be selected to in a specific position in proximity to first point **704** such that changes in properties **714** caused by the performance of action **710** at first point **704** can be measured by sensors **716** to generate sensor data **722.**

In this and some other examples, second point **706** is a location in composite material manufacturing system **708** at which another set of actions **720** can be performed on portion **700** of unconsolidated composite material **702.** The set of actions **720** performed at second point **706** can change properties **714.**

As result, the set of sensors **716** at sensor location **718** between first point **704** and second point **706** can measure properties **714** present after the performance of actions **710** on portion **700** at first point **704.** This measurement can be made at time intervals **715.** For example, a measurement can be made every second. Knowing the speed at which unconsolidated composite material **702** moves, properties for specific portions **719,** such as portion **700** can be identified. The set of sensors **716** can generate sensor data **722** from those measurements of the set of properties **714** for use in determining predicted properties for portion **700** of unconsolidated composite material **702** that will be present when the manufacturing of portion **700** of unconsolidated composite material **702** has been completed.

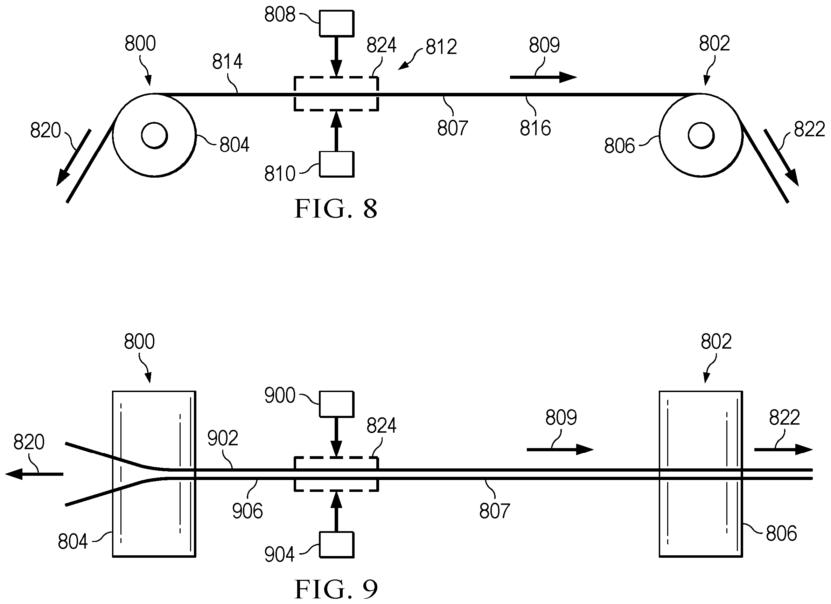

With reference now to **Figure 8****,** an illustration of the side view of a portion of a composite material manufacturing system is depicted in accordance with an illustrative example. In this and some other examples, point 1 **800** and point 2 **802** are examples of first point **704** and second point **706 in** **Figure 7****.** Roller **804** is located at point 1 **800** and roller **806** is located at point 2 **802.**

An unconsolidated composite material in the form of prepreg **807** moves in the direction of arrow **809** from point 1 **800** to point 2 **802.** The speed of movement is distance/time, such as feet per second. Knowing the speed that prepreg **807** moves enables knowing the length and location of portion **824** as that portion moves within the composite material manufacturing system.

In this and some other examples, sensor **808** and sensor **810** are positioned at sensor location **812.** At sensor location **812,** sensor **808** is positioned on top side **814** of prepreg **807.** Sensor **808** is positioned on bottom side **816** of prepreg **807.**

In this and some other examples, tension **820** at point 1 **800** and tension **822** at point 2 **802** are present. Additionally, roller **804** applies heat and pressure to prepreg **807** as prepreg **807** travels over roller **804.** Sensor **808** and sensor **810** can make real time measurements of thickness and reflectance of portion **824** of prepreg **807** at sensor location **812** that results from applying tension, heat, and pressure to prepreg **807** to generate sensor data.

Turning to **Figure 9****,** an illustration of the top view of a portion of a composite material manufacturing system is depicted in accordance with an illustrative example. In this and some other examples, a different positioning of sensors is present as compared to the example in **Figure 8****.** As depicted in this top view, sensor **900** is positioned on first side **902** of prepreg **807** at sensor location **812.** Sensor **904** is position on second side **906** of prepreg **807** at sensor location **812,** which is opposite to first side **902.**

In this and some other examples, tension **820** at point 1 **800** and tension **822** at point 2 **802** are present. Additionally, roller **804** applies heat and pressure to prepreg **807** as prepreg **807** travels over roller **804.**

As depicted, sensor **900** and sensor **904** can make real time measurements of width, conductance, and capacitance of portion **824** of prepreg **807** to generate sensor data. The sensor data of the electrical measurements can be used to determine properties for portion **824** of prepreg **807** such as the amount resin absorption and width.

With the sensor data from at least one of sensor location **812** or other sensor locations, predictions of the properties for portion **824** of prepreg **807** can be made for portion **824** when the manufacturing of portion **824** of prepreg **807** is complete, such as when portion **824** of prepreg **807** is wound onto a prepreg roll. For example, the sensor data can be used to predict properties that portion **824** of prepreg **807** will have such as fiber areal weight (FAW) and water pickup

The illustration of the points, sensor location, and sensors are provided as an example of one implementation and not meant to limit the manner in which other illustrative examples can be implemented. For example, in other illustrative examples, only a single sensor may be present, or 3 or more sensors may be present. Further, the additional sensors can be at sensor location **812** or another sensor location between point 1 **800** and point 2 **802.**

As yet another example, point 1 **800** and point 2 **802** can be locations for other components that perform actions on prepreg **807** other than roller **804** and roller **806.** For example, a collar, a spreader bar, a comb, a collar, a nip, a rewinder, or other component can be at point 1 **800** and point 2 **802.**

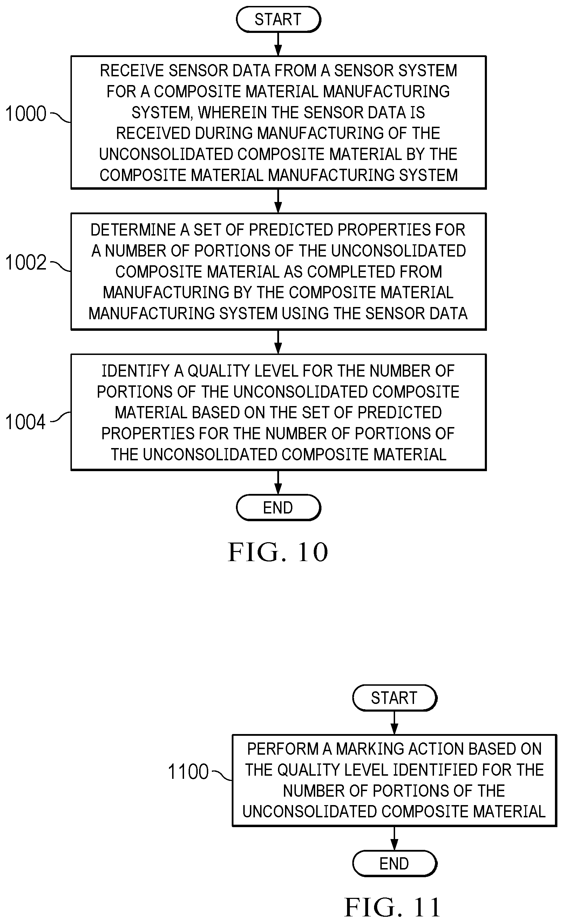

Turning to **Figure 10****,** an illustration of a flowchart of a process for identifying quality levels for portions of an unconsolidated composite material is depicted in accordance with an illustrative example. The process in **Figure 10** can be implemented in hardware, software, or both. When implemented in software, the process can take the form of program code that is run by one of more processor units located in one or more hardware devices in one or more computer systems. For example, the process can be implemented in composite material manager **212** in computer system **210 in** **Figure 2****.**

As depicted, the process begins by receiving sensor data from a sensor system for a composite material manufacturing system, wherein the sensor data is received during manufacturing of the unconsolidated composite material by the composite material manufacturing system (operation **1000).** The process determines a set of predicted properties for a number of portions of the unconsolidated composite material as completed from manufacturing by the composite material manufacturing system using the sensor data (operation **1002).** The process identifies a quality level for the number of portions of the unconsolidated composite material based on the set of predicted properties for the number of portions of the unconsolidated composite material (operation **1004).** The process terminates thereafter.

**Figure 11****,** an illustration of a flowchart of a process for marking an unconsolidated composite material is depicted in accordance with an illustrative example. The operations in this figure are examples of additional operations that can be used within the operations in the process in **Figure 10****.**

As depicted, the process performs a marking action based on the quality level identified for the number of portions of the unconsolidated composite material (operation **1100).** The process terminates thereafter.

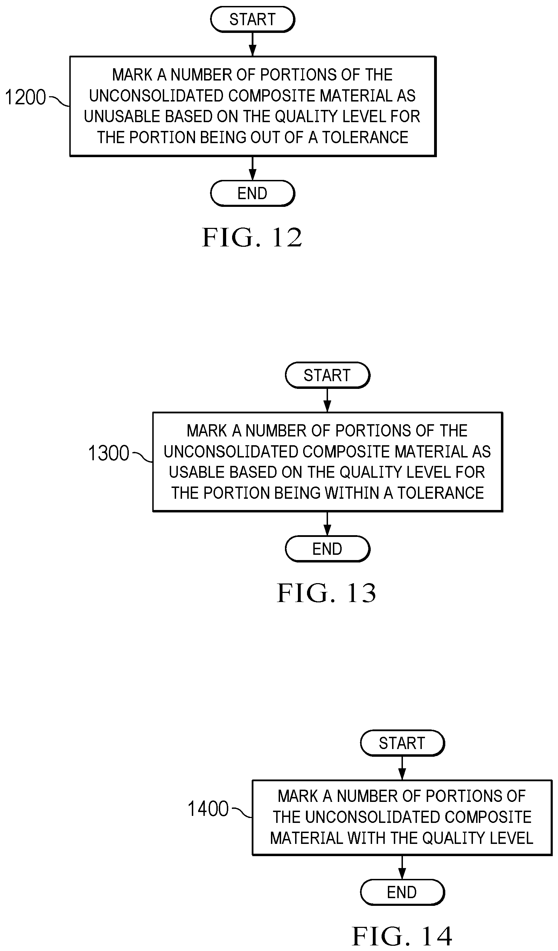

Next in **Figure 12****,** an illustration of a flowchart of a process for marking an out-of-tolerance portion of an unconsolidated composite material as unusable is depicted in accordance with an illustrative example. The operation in this figure is an example of an implementation of operation **1100 in** **Figure 11****.**

The process marks a number of portions of the unconsolidated composite material in the number of portions of the unconsolidated composite material as unusable based on the quality level for the portion being out of a tolerance (operation **1200).** The process terminates thereafter.

With reference next to **Figure 13****,** an illustration of a flowchart of a process for marking an in-tolerance portion of an unconsolidated composite material as usable is depicted in accordance with an illustrative example. The operation in this figure is an example of an implementation of operation **1100 in** **Figure 11****.**

The process marks a number of portions of the unconsolidated composite material in the number of portions of the unconsolidated composite material as usable based on the quality level for the portion being within a tolerance (operation **1300).** The process terminates thereafter.

Turning to **Figure 14****,** an illustration of a flowchart of a process for marking a number of portions of an unconsolidated composite material with a quality level is depicted in accordance with an illustrative example. The operation in this figure is an example of an implementation of operation **1100 in** **Figure 11****.**

The process marks a portion of the unconsolidated composite material in the number of portions of the unconsolidated composite material with the quality level (operation **1400).** The process terminates thereafter.

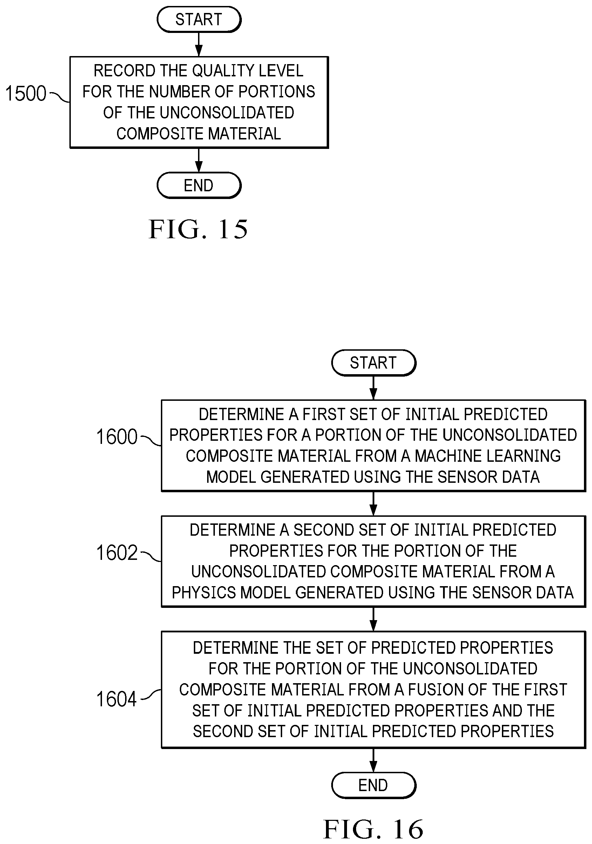

With reference now to **Figure 15****,** an illustration of a flowchart of a process for recording a quality level for portions of an unconsolidated composite material is depicted in accordance with an illustrative example. The operation in this figure is an example of an implementation of operation **1100 in** **Figure 11****.**

The process records the quality level for the number of portions of the unconsolidated composite material (operation **1500).** The process terminates thereafter.

With reference to **Figure 16****,** an illustration of a flowchart of a process for determining predicted properties for a portion of an unconsolidated composite material is depicted in accordance with an illustrative example. The operations in this figure are an example of one implementation of operation **1002 in** **Figure 10****.**

As depicted, the process begins by determining a first set of initial predicted properties for the portion of the unconsolidated composite material from a machine learning model generated using the sensor data (operation **1600).** The process determines a second set of initial predicted properties for the portion of the unconsolidated composite material from a physics model generated using the sensor data (operation **1602).** The process determines the set of predicted properties for the portion of the unconsolidated composite material from a fusion of the first set of initial predicted properties and the second set of initial predicted properties (operation **1604).** The process terminates thereafter.

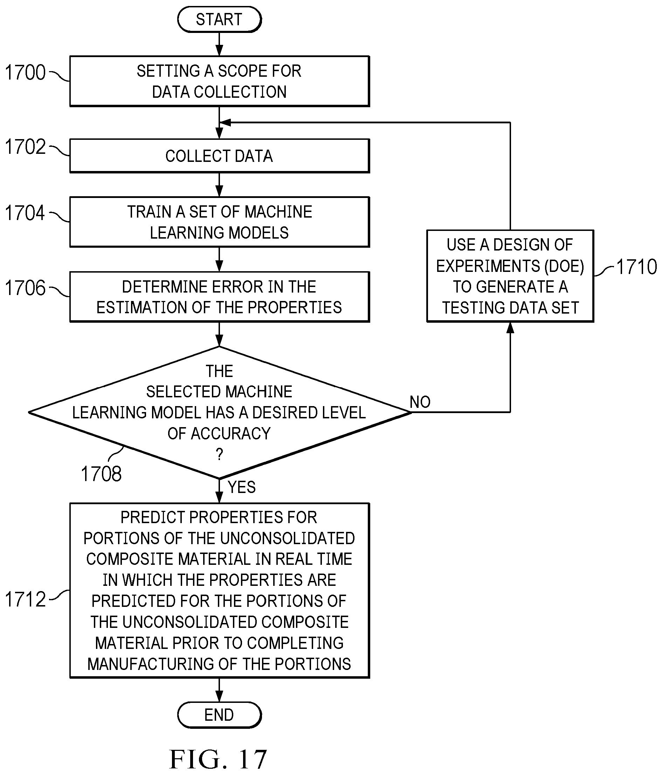

Turning next to **Figure 17****,** an illustration of a flowchart of a process for training machine learning model to predict properties for an unconsolidated composite material prior to completing fabrication of the unconsolidated composite material is depicted in accordance with an illustrative example. The process in **Figure 17** can be implemented in hardware, software, or both. When implemented in software, the process can take the form of program code that is run by one of more processor units located in one or more hardware devices in one or more computer systems. For example, the process can be implemented in composite material manager **212** in computer system **210 in** **Figure 2****.**

The process begins by setting a scope for data collection (operation **1700).** In operation **1700,** the scope is set such that the data is collected with sufficient resolution repetition for training, validating testing models.

The process collects data (operation **1702).** In this operation, historical data is collected from the manufacturing of the unconsolidated composite materials. This data can include sensor data, upstream data, and the inspection data. The inspection data is generated by sample testing the manufactured unconsolidated composite material to determine the properties of interest. These properties of interest are used to evaluate the quality of the unconsolidated composite material and whether it is suitable for particular uses.

The process trains a set of machine learning models (operation **1704).** In operation **1704,** one or more model types can be selected for training. When more than one type of model is selected, and evaluation can be performed to see which machine learning model provides the most accurate predictions.

In operation **1704,** properties and parameters are selected for use in training. The properties are properties of unconsolidated composite materials that are of interest in determining the quality and whether the unconsolidated composite material is suitable for use. The parameters are selected as parameters that have a correlation or effect on the properties selected. In other words, when the parameters change, those changes can have an effect on the properties that result for the unconsolidated composite material.

Operation **1704** can also include hyperparameter optimization in which hyperparameters in the model can be set as parameters or variables that are set before actually optimizing the parameters or variables selected for training the model. Hyperparameters can include, for example, model selection.

The process determines error in the estimation of the properties (operation **1706).** In operation **1706,** the validation data can be used to determine the error occurring in predicting properties.

The process then determines whether the selected machine learning model has a desired level of accuracy (operation **1708).** This desired level accuracy can be tested using a portion of the historical data that is partitioned off as testing data. If the level of accuracy is not present, the process uses a design of experiments (DOE) to generate a testing data set (operation **1710).** The process then returns to operation **1702** to collect data using the data generation approach generated by the design of experiments. The design of experiments is a statistical method to guide the execution of experiments to generate training.

With reference again to operation **1708,** if the selected machine learning model has a desired level of accuracy, then the process predicts properties for portions of the unconsolidated composite material in real time in which the properties are predicted for the portions of the unconsolidated composite material prior to completing manufacturing of the portions (operation **1712).** The process terminates thereafter.

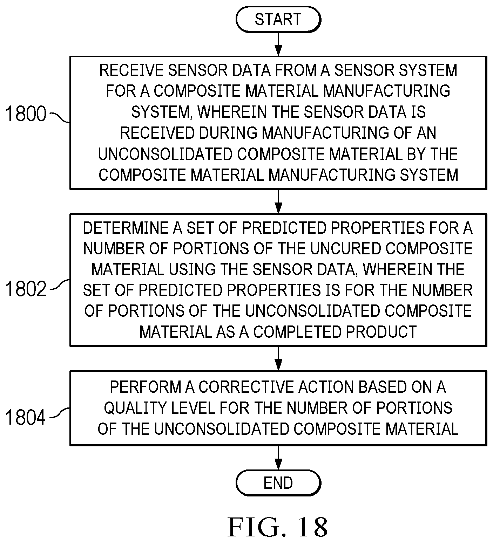

Turning to **Figure 18****,** an illustration of a flowchart of a process for performing a corrective action for portions of an unconsolidated composite material is depicted in accordance with an illustrative example. The process in **Figure 18** can be implemented in hardware, software, or both. When implemented in software, the process can take the form of program code that is run by one of more processor units located in one or more hardware devices in one or more computer systems. For example, the process can be implemented in composite material manager **212** in computer system **210** in **Figure 2****.**

As depicted, the process begins by receiving sensor data from a sensor system for a composite material manufacturing system, wherein the sensor data is received during manufacturing of an unconsolidated composite material by the composite material manufacturing system (operation **1800).** The process determines a set of predicted properties for a number of portions of the unconsolidated composite material using the sensor data, wherein the set of predicted properties is for the number of portions of the unconsolidated composite material as a completed product (operation **1802).** The process performs a corrective action based on a quality level for the number of portions of the unconsolidated composite material (operation **1804).** The process terminates thereafter.

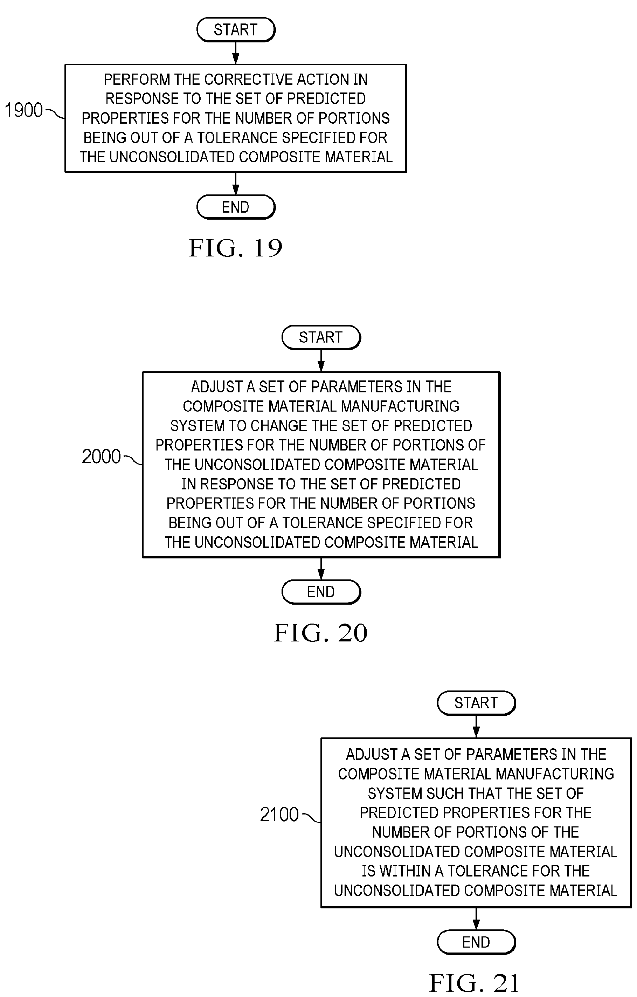

Turning to **Figure 19****,** an illustration of a flowchart of a process for performing a corrective action for out-of-tolerance portions of an unconsolidated composite material is depicted in accordance with an illustrative example. The operation in this figure is an example of an implementation of operation **1804** in **Figure 18****.**

As depicted, the process performs the corrective action in response to the set of predicted properties for the number of portions being out of a tolerance specified for the unconsolidated composite material (operation **1900).** The process terminates thereafter.

Turning next to **Figure 20****,** an illustration of a flowchart of a process for adjusting parameters for out-of-tolerance portions of an unconsolidated composite material is depicted in accordance with an illustrative example. The operation in this figure is an example of an implementation of operation **1804** in **Figure 18****.**

As depicted, the process adjusts a set of parameters in the composite material manufacturing system to change the set of predicted properties for the number of portions of the unconsolidated composite material in response to the set of predicted properties for the number of portions being out of a tolerance specified for the unconsolidated composite material (operation **2000).** The process terminates thereafter.

With reference to **Figure 21****,** an illustration of a flowchart of a process for adjusting parameters for in-tolerance portions of an unconsolidated composite material is depicted in accordance with an illustrative example. The operation in this figure is an example of an implementation of operation **1804** in **Figure 18****.**

As depicted, the process adjusts a set of parameters in the composite material manufacturing system such that the set of predicted properties for the number of portions of the unconsolidated composite material is within a tolerance for the unconsolidated composite material (operation **2100).** The process terminates thereafter.

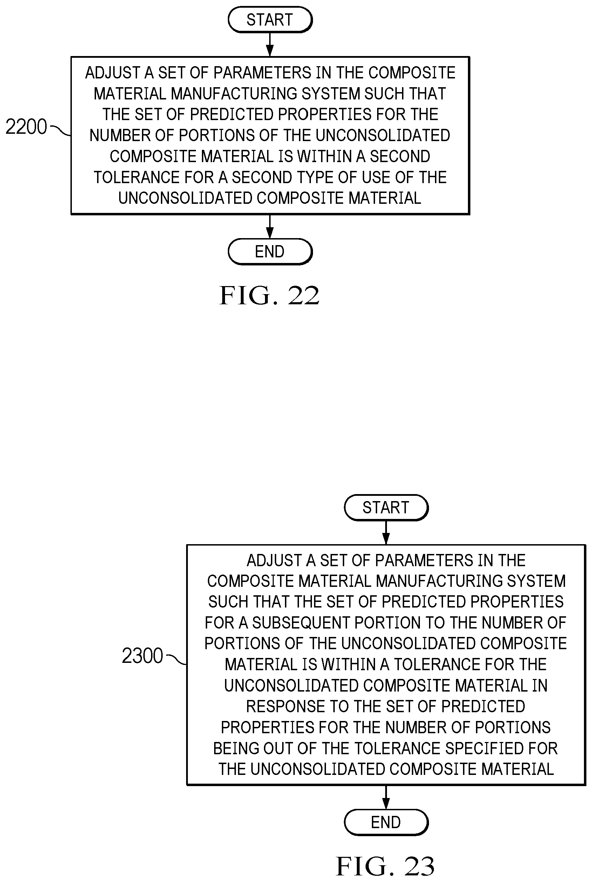

Turning next to **Figure 22****,** an illustration of a flowchart of a process for adjusting parameters such that portions of an unconsolidated composite material are within tolerance is depicted in accordance with an illustrative example. The operation in this figure is an example of an implementation of operation **1804** in **Figure 18****.**

As depicted, the process adjusts a set of parameters in the composite material manufacturing system such that the set of predicted properties for the number of portions of the unconsolidated composite material is within a second tolerance for a second type of use of the unconsolidated composite material (operation **2200).** The process terminates thereafter.

With reference to **Figure 23****,** an illustration of a flowchart of a process for adjusting parameters for a subsequent portion of an unconsolidated composite material is depicted in accordance with an illustrative example. The operation in this figure is an example of an implementation of operation **1804** in **Figure 18****.**

As depicted, the process adjusts a set of parameters in the composite material manufacturing system such that the set of predicted properties for a subsequent portion to the number of portions of the unconsolidated composite material is within a tolerance for the unconsolidated composite material in response to the set of predicted properties for the number of portions being out of the tolerance specified for the unconsolidated composite material (operation **2300).** The process terminates thereafter.

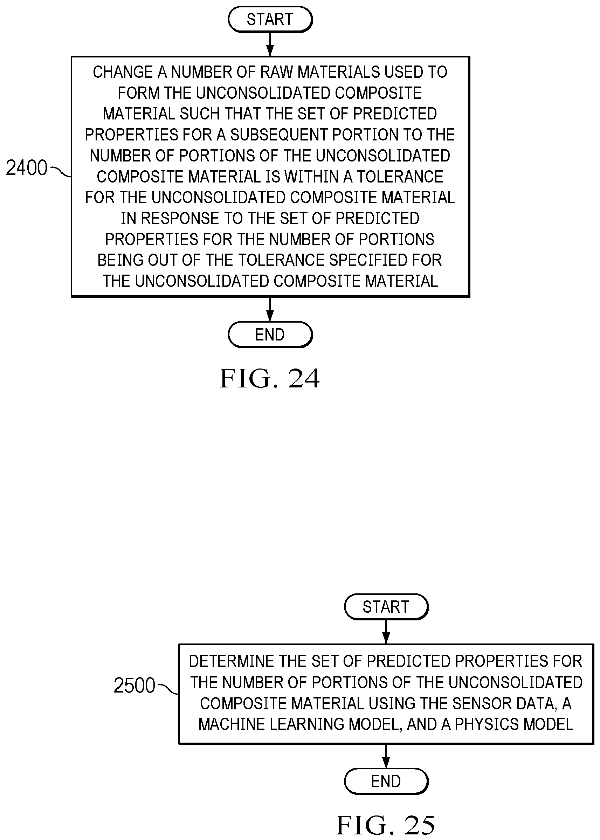

Turning to **Figure 24****,** an illustration of a flowchart of a process for changing raw materials is depicted in accordance with an illustrative example. The operation in this figure is an example of an implementation of operation **1804** in **Figure 18****.**

As depicted, the process changes a number of raw materials used to form the unconsolidated composite material such that the set of predicted properties for a subsequent portion to the number of portions of the unconsolidated composite material is within a tolerance for the unconsolidated composite material in response to the set of predicted properties for the number of portions being out of the tolerance specified for the unconsolidated composite material (operation **2400).** The process terminates thereafter.

Turning next to **Figure 25****,** an illustration of a flowchart of a process for determining predicted properties of an unconsolidated composite material is depicted in accordance with an illustrative example. The operations in this figure are examples of additional operations that can be used within the operations in the process in **Figure 18****.**

As depicted, the process determines the set of predicted properties for the number of portions of the unconsolidated composite material using the sensor data, a machine learning model, and a physics model (operation **2500).** The process terminates thereafter.

The flowcharts and block diagrams in the different depicted examples illustrate the architecture, functionality, and operation of some possible implementations of apparatuses and methods. In this regard, each block in the flowcharts or block diagrams can represent at least one of a module, a segment, a function, or a portion of an operation or step. For example, one or more of the blocks can be implemented as program code, hardware, or a combination of the program code and hardware. When implemented in hardware, the hardware can, for example, take the form of integrated circuits that are manufactured or configured to perform one or more operations in the flowcharts or block diagrams. When implemented as a combination of program code and hardware, the implementation may take the form of firmware. Each block in the flowcharts or the block diagrams can be implemented using special purpose hardware systems that perform the different operations or combinations of special purpose hardware and program code run by the special purpose hardware.

In some alternative implementations, the function or functions noted in the blocks may occur out of the order noted in the figures. For example, in some cases, two blocks shown in succession may be performed concurrently or substantially concurrently, or the blocks may sometimes be performed in the reverse order, depending upon the functionality involved. Also, other blocks may be added in addition to the illustrated blocks in a flowchart or block diagram.

Turning now to **Figure 26****,** an illustration of a block diagram of a data processing system is depicted in accordance with an illustrative example. Data processing system **2600** can be used to implement server computer **104,** server computer **106,** client devices **110,** in **Figure 1****.** Data processing system **2600** can also be used to implement computer system **210** in **Figure 2****.** In this and some other examples, data processing system **2600** includes communications framework **2602,** which provides communications between processor unit **2604,** memory **2606,** persistent storage **2608,** communications unit **2610,** input/output (I/O) unit **2612,** and display **2614.** In this and some other examples, communications framework **2602** takes the form of a bus system.

Processor unit **2604** serves to execute instructions for software that can be loaded into memory **2606.** Processor unit **2604** includes one or more processors. For example, processor unit **2604** can be selected from at least one of a multicore processor, a central processing unit (CPU), a graphics processing unit (GPU), a physics processing unit (PPU), a digital signal processor (DSP), a network processor, or some other suitable type of processor. Further, processor unit **2604** can may be implemented using one or more heterogeneous processor systems in which a main processor is present with secondary processors on a single chip. As another illustrative example, processor unit **2604** can be a symmetric multi-processor system containing multiple processors of the same type on a single chip.

Memory **2606** and persistent storage **2608** are examples of storage devices **2616.** A storage device is any piece of hardware that is capable of storing information, such as, for example, without limitation, at least one of data, program code in functional form, or other suitable information either on a temporary basis, a permanent basis, or both on a temporary basis and a permanent basis. Storage devices **2616** may also be referred to as computer-readable storage devices in these illustrative examples. Memory **2606,** in these and some other examples, can be, for example, a random-access memory or any other suitable volatile or nonvolatile storage device. Persistent storage **2608** can take various forms, depending on the particular implementation.

For example, persistent storage **2608** may contain one or more components or devices. For example, persistent storage **2608** can be a hard drive, a solid-state drive (SSD), a flash memory, a rewritable optical disk, a rewritable magnetic tape, or some combination of the above. The media used by persistent storage **2608** also can be removable. For example, a removable hard drive can be used for persistent storage **2608.**

Communications unit **2610,** in these and some other examples, provides for communications with other data processing systems or devices. In these and some other examples, communications unit **2610** is a network interface card.

Input/output unit **2612** allows for input and output of data with other devices that can be connected to data processing system **2600.** For example, input/output unit **2612** can provide a connection for user input through at least one of a keyboard, a mouse, or some other suitable input device. Further, input/output unit **2612** can send output to a printer. Display **2614** provides a mechanism to display information to a user.

Instructions for at least one of the operating system, applications, or programs can be located in storage devices **2616,** which are in communication with processor unit **2604** through communications framework **2602.** The processes of the different example can be performed by processor unit **2604** using computer-implemented instructions, which can be located in a memory, such as memory **2606.**

These instructions are program instructions and are also referred to as program code, computer usable program code, or computer-readable program code that can be read and executed by a processor in processor unit **2604.** The program code in the different examples can be embodied on different physical or computer-readable storage media, such as memory **2606** or persistent storage **2608.**

Program code **2618** is located in a functional form on computer-readable media **2620** that is selectively removable and can be loaded onto or transferred to data processing system **2600** for execution by processor unit **2604.** Program code **2618** and computer-readable media **2620** form computer program product **2622** in these and some other examples. In some examples including the illustrative example, computer-readable media **2620** is computer-readable storage media **2624.**

Computer-readable storage media **2624** is a physical or tangible storage device used to store program code **2618** rather than a media that propagates or transmits program code **2618.** Computer readable storage media **2624,** as used herein, is not to be construed as being transitory signals *per se,* such as radio waves or other freely propagating electromagnetic waves, electromagnetic waves propagating through a waveguide or other transmission media (e.g., light pulses passing through a fiber-optic cable), or electrical signals transmitted through a wire.

Alternatively, program code **2618** can be transferred to data processing system **2600** using a computer-readable signal media. The computer-readable signal media are signals and can be, for example, a propagated data signal containing program code **2618.** For example, the computer-readable signal media can be at least one of an electromagnetic signal, an optical signal, or any other suitable type of signal. These signals can be transmitted over connections, such as wireless connections, optical fiber cable, coaxial cable, a wire, or any other suitable type of connection.

Further, as used herein, "computer-readable media **2620"** can be singular or plural. For example, program code **2618** can be located in computer-readable media **2620** in the form of a single storage device or system. In some other examples, program code **2618** can be located in computer-readable media **2620** that is distributed in multiple data processing systems. In other words, some instructions in program code **2618** can be located in one data processing system while other instructions in program code **2618** can be located in one data processing system. For example, a portion of program code **2618** can be located in computer-readable media **2620** in a server computer while another portion of program code **2618** can be located in computer-readable media **2620** located in a set of client computers.

The different components illustrated for data processing system **2600** are not meant to provide architectural limitations to the manner in which different examples can be implemented. In some illustrative examples, one or more of the components may be incorporated in or otherwise form a portion of, another component. For example, memory **2606,** or portions thereof, can be incorporated in processor unit **2604** in some illustrative examples. The different illustrative examples can be implemented in a data processing system including components in addition to or in place of those illustrated for data processing system **2600.** Other components shown in **Figure 26** can be varied from the illustrative examples shown. The different examples can be implemented using any hardware device or system capable of running program code **2618.**

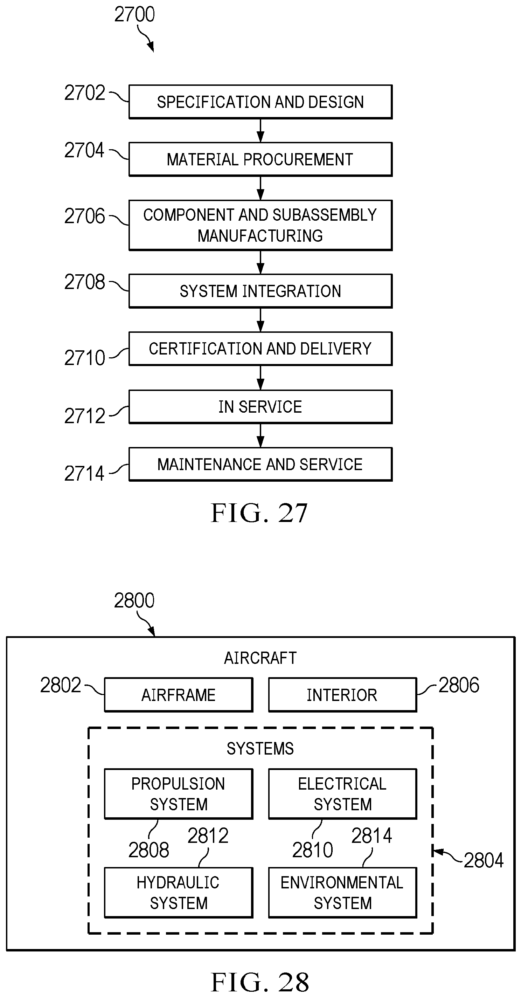

Illustrative examples of the disclosure may be described in the context of aircraft manufacturing and service method **2700** as shown in **Figure 27** and aircraft **2800** as shown in **Figure 28****.** Turning first to **Figure 27****,** an illustration of an aircraft manufacturing and service method is depicted in accordance with an illustrative example. During pre-production, aircraft manufacturing and service method **2700** may include specification and design **2702** of aircraft **2800 in** **Figure 28** and material procurement **2704.**

During production, component and subassembly manufacturing **2706** and system integration **2708** of aircraft **2800 in** **Figure 28** takes place. Thereafter, aircraft **2800 in** **Figure 28** can go through certification and delivery **2710** in order to be placed in service **2712.** While in service **2712** by a customer, aircraft **2800** in **Figure 28** is scheduled for routine maintenance and service **2714,** which may include modification, reconfiguration, refurbishment, and other maintenance or service.

Each of the processes of aircraft manufacturing and service method **2700** may be performed or carried out by a system integrator, a third party, an operator, or some combination thereof. In these and some other examples, the operator may be a customer. For the purposes of this description, a system integrator may include, without limitation, any number of aircraft manufacturers and major-system subcontractors; a third party may include, without limitation, any number of vendors, subcontractors, and suppliers; and an operator may be an airline, a leasing company, a military entity, a service organization, and so on.

With reference now to **Figure 28****,** an illustration of an aircraft is depicted in which an illustrative example may be implemented. In some examples including this example, aircraft **2800** is produced by aircraft manufacturing and service method **2700 in** **Figure 27** and may include airframe **2802** with plurality of systems **2804** and interior **2806.** Examples of systems **2804** include one or more of propulsion system **2808,** electrical system **2810,** hydraulic system **2812,** and environmental system **2814.** Any number of other systems may be included. Although an aerospace example is shown, different illustrative examples may be applied to other industries, such as the automotive industry.

Apparatuses and methods embodied herein may be employed during at least one of the stages of aircraft manufacturing and service method **2700** in **Figure 27****.**

In some illustrative examples, components or subassemblies produced in component and subassembly manufacturing **2706** in **Figure 27** can be fabricated or manufactured in a manner similar to components or subassemblies produced while aircraft **2800** is in service **2712** in **Figure 27****.** In yet other examples, one or more apparatus examples, method examples, or a combination thereof can be utilized during production stages, such as component and subassembly manufacturing **2706** and system integration **2708** in **Figure 27****.** One or more apparatus examples, method examples, or a combination thereof may be utilized while aircraft **2800** is in service **2712,** during maintenance and service **2714** in **Figure 27****,** or both. The use of a number of the different illustrative examples may expedite or substantially expedite the assembly of aircraft **2800,** reduce the cost of aircraft **2800,** or both expedite the assembly of aircraft **2800** and reduce the cost of aircraft **2800.** Increased quality in manufacturing unconsolidated composite materials during at least one of component and subassembly manufacturing **2706** or maintenance and service **2714** can at least one of reduce the amount of time needed or cost in manufacturing unconsolidated composite materials. With the ability to determine which portions of unconsolidated composite materials meet desired quality levels, less discarding or rework of unconsolidated composite materials can occur.

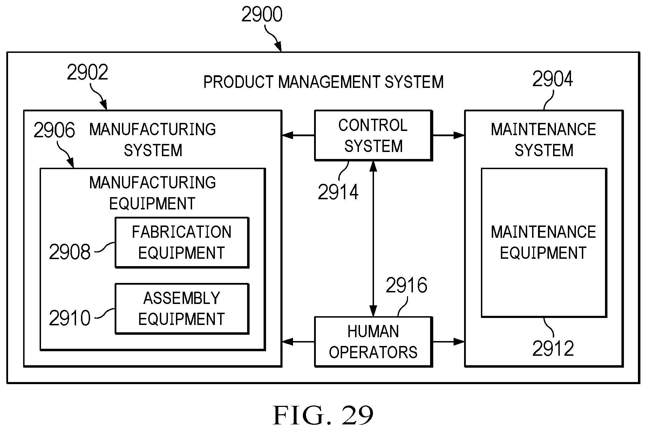

Turning now to **Figure 29****,** an illustration of a block diagram of a product management system is depicted in accordance with an illustrative example. Product management system **2900** is a physical hardware system. In some examples including this illustrative example, product management system **2900** includes at least one of manufacturing system **2902** or maintenance system **2904.**

Manufacturing system **2902** is configured to manufacture products, such as aircraft **2800** in **Figure 28****.** As depicted, manufacturing system **2902** includes manufacturing equipment **2906.** Manufacturing equipment **2906** includes at least one of fabrication equipment **2908** or assembly equipment **2910.**

Fabrication equipment **2908** is equipment that used to fabricate components for parts used to form aircraft **2800** in **Figure 28****.** For example, fabrication equipment **2908** can include machines and tools. These machines and tools can be at least one of a drill, a hydraulic press, a furnace, an autoclave, a mold, a composite tape laying machine, an automated fiber placement (AFP) machine, a vacuum system, a robotic pick and place system, a flatbed cutting machine, a laser cutter, a computer numerical control (CNC) cutting machine, a lathe, or other suitable types of equipment. Fabrication equipment **2908** can be used to fabricate at least one of metal parts, composite parts, semiconductors, circuits, fasteners, ribs, skin panels, spars, antennas, or other suitable types of parts.

Assembly equipment **2910** is equipment used to assemble parts to form aircraft **2800** in **Figure 28****.** In particular, assembly equipment **2910** is used to assemble components and parts to form aircraft **2800** in **Figure 28****.** Assembly equipment **2910** also can include machines and tools. These machines and tools may be at least one of a robotic arm, a crawler, a faster installation system, a rail-based drilling system, or a robot. Assembly equipment **2910** can be used to assemble parts such as seats, horizontal stabilizers, wings, engines, engine housings, landing gear systems, and other parts for aircraft **2800** in **Figure 28****.**

In this illustrative example, maintenance system **2904** includes maintenance equipment **2912.** Maintenance equipment **2912** can include any equipment needed to perform maintenance on aircraft **2800** in **Figure 28****.** Maintenance equipment **2912** may include tools for performing different operations on parts on aircraft **2800** in **Figure 28****.** These operations can include at least one of disassembling parts, refurbishing parts, inspecting parts, reworking parts, manufacturing replacement parts, or other operations for performing maintenance on aircraft **2800** in **Figure 28****.** These operations can be for routine maintenance, inspections, upgrades, refurbishment, or other types of maintenance operations.

In some examples including the illustrative example, maintenance equipment **2912** may include ultrasonic inspection devices, x-ray imaging systems, vision systems, drills, crawlers, and other suitable devices. In some cases, maintenance equipment **2912** can include fabrication equipment **2908,** assembly equipment **2910,** or both to produce and assemble parts that needed for maintenance.

Product management system **2900** also includes control system **2914.** Control system **2914** is a hardware system and may also include software or other types of components. Control system **2914** is configured to control the operation of at least one of manufacturing system **2902** or maintenance system **2904.** In particular, control system **2914** can control the operation of at least one of fabrication equipment **2908,** assembly equipment **2910,** or maintenance equipment **2912.**

The hardware in control system **2914** can be implemented using hardware that may include computers, circuits, networks, and other types of equipment. The control may take the form of direct control of manufacturing equipment **2906.** For example, robots, computer-controlled machines, and other equipment can be controlled by control system **2914.** In other illustrative examples, control system **2914** can manage operations performed by human operators **2916** in manufacturing or performing maintenance on aircraft **2800.** For example, control system **2914** can assign tasks, provide instructions, display models, or perform other operations to manage operations performed by human operators **2916.** In these and some other examples, composite material manager **212** in **Figure 2** can be implemented in control system **2914** to manage manufacturing of unconsolidated composite materials for use in at least one of the manufacturing or maintenance of aircraft **2800** in **Figure 28****.** These unconsolidated composite materials can be used to manufacture composite parts or to rework composite parts in at least one of least one of the manufacturing or maintenance of aircraft **2800** in **Figure 28****.**

In the different illustrative examples, human operators **2916** can operate or interact with at least one of manufacturing equipment **2906,** maintenance equipment **2912,** or control system **2914.** This interaction can occur to manufacture aircraft **2800** in **Figure 28****.**

Of course, product management system **2900** may be configured to manage other products other than aircraft **2800** in **Figure 28****.** Although product management system **2900** has been described with respect to manufacturing in the aerospace industry, product management system **2900** can be configured to manage products for other industries. For example, product management system **2900** can be configured to manufacture products for the automotive industry as well as any other suitable industries.

Thus, the illustrative examples provide a method, apparatus, system, and computer program product for predicting properties that unconsolidated composite materials will have upon completion of manufacturing the unconsolidated composite materials while the manufacturing process occurs. Further, the illustrative examples can also perform corrective actions using these predictions to change the properties that the unconsolidated composite materials will have as completed from manufacturing to form a completed product.

In the different illustrative examples, a composite material manager performs operations to determine a set of predicted properties for a number of portions of the unconsolidated composite material has completed from manufacturing. In other words, the prediction is for the portions of the uncured composite material when the manufacturing of the number of portions of uncured composite materials is completed to form a completed product.

As a result, a reduction in at least one of expense or the amount of time needed to manufacture unconsolidated composite materials can be realized using composite material manager 212 to predict quality level portions of unconsolidated composite material. Further the reduction in the can occur by performing corrective actions during manufacturing of the unconsolidated composite material such that the additional amounts of unconsolidated material that are in tolerance occur. As result, additional manufacturing, reworking, or discarding of unconsolidated composite material can be reduced.

The description of the different illustrative examples has been presented for purposes of illustration and description and is not intended to be exhaustive or limited to the examples in the form disclosed. The different illustrative examples describe components that perform actions or operations. In an illustrative example, a component can be configured to perform the action or operation described. For example, the component can have a configuration or design for a structure that provides the component an ability to perform the action or operation that is described in the illustrative examples as being performed by the component. Further, To the extent that terms "includes", "including", "has", "contains", and variants thereof are used herein, such terms are intended to be inclusive in a manner similar to the term "comprises" as an open transition word without precluding any additional or other elements.

## Claims

1. A computer-implemented method for manufacturing an unconsolidated composite material, the method comprising:
receiving sensor data (134, 218, 722) from a sensor system (220) for a composite material manufacturing system (206, 500, 600, 708), wherein the sensor data (134, 218, 722) is about at least one of the unconsolidated composite material (204) or the composite material manufacturing system (208) during the operation of composite material manufacturing system (208) and is received during manufacturing of an unconsolidated composite material (204, 702) by the composite material manufacturing system (206, 500, 600, 708);
determining a set of predicted properties (224) for a number of portions (226) of the unconsolidated composite material (204, 702) as completed from manufacturing by the composite material manufacturing system (206, 500, 600, 708) using the sensor data (134, 218, 722) and a machine learning model (138, 236), wherein:
the machine learning model is trained to predict properties (228) for the unconsolidated composite material (204) using sensor data (218),
the machine learning model (138, 236) has been trained using a training dataset comprising historical sensor data (242) generated from previously manufacturing the unconsolidated composite material (204) and labels in the form of historical properties (244), and
the historical properties (244) are determined from measurements made of unconsolidated composite material (204) at different locations in the composite material manufacturing system (206), and include properties (228) of unconsolidated composite material (204) when manufacturing of unconsolidated composite material (204) has been completed; and
identifying a quality level (230) for the number of portions (226) of the unconsolidated composite material (204, 702) based on the set of predicted properties (224) for the number of portions (226) of the unconsolidated composite material (204, 702).

2. The method of claim 1, comprising:
performing a marking action (300) based on the quality level (230) identified for the number of portions (226) of the unconsolidated composite material (204, 702) and, optionally:
marking at least some of the number of portions (226) of the unconsolidated composite material (204, 702) as unusable (304) based on the quality level (230) for those portions (302, 700, 824) being out of a tolerance (312); and/or
marking at least some of the number of portions (226) of the unconsolidated composite material (204, 702) as usable (306) based on the quality level (230) for those portion (302, 700, 824) being within a tolerance (312).

3. The method of claim 2, wherein performing the marking action (300) comprises:
marking the number of portions (226) of the unconsolidated composite material (204, 702) with the quality level (230);
and/or
recording the quality level (230) for the number of portions (226) of the unconsolidated composite material (204, 702).

4. The method of any preceding claim, wherein the composite material manufacturing system (206, 500, 600, 708) is a carbon fiber prepreg coating line, a reinforcing fiber resin impregnation line, a resin mix process system, a resin filming line, a prepreg system, or a slitting process system.

5. The method of any preceding claim wherein a training dataset for the machine learning model comprises the historical sensor data, and:
(a) labels in the form of historical properties (244), wherein, optionally, the historical properties (244) are determined from measurements made of unconsolidated composite material (204) at different locations in the composite material manufacturing system (206); and, optionally,
(b) historical upstream information (245), wherein, optionally, the historical upstream information (245) is associated with historical sensor data (242) generated when manufacturing an unconsolidated composite material in which the manufacturing process includes historical upstream information, wherein the historical upstream information (245) optionally comprises information about components or settings used in manufacturing unconsolidated composite materials.

6. The method of any preceding claim, further comprising determining the set of predicted properties (224) for a portion (302, 700, 824) of the unconsolidated composite material (204, 702) using the sensor data (134, 218, 722), the machine learning model (138, 236), and a physics model (239) and, optionally:
determining a first set of initial predicted properties (251) for the portion (302, 700, 824) of the unconsolidated composite material (204, 702) from the machine learning model (138, 236) generated using the sensor data (134, 218, 722);
determining a second set of initial predicted properties (251) for the portion (302, 700, 824) of the unconsolidated composite material (204, 702) from the physics model (239) generated using the sensor data (134, 218, 722); and
determining the set of predicted properties (224) for the portion (302, 700, 824) of the unconsolidated composite material (204, 702) from a fusion of the first set of initial predicted properties (251) and the second set of initial predicted properties (251).

7. The method of any preceding claim, wherein the unconsolidated composite material (204, 702) is a prepreg (648, 712, 807), an aerospace-grade prepreg, a thermoset prepreg, a thermoplastic prepreg, a woven fabric prepreg, a fiber tow prepreg, a tow prepreg, unidirectional tape prepreg, a resin coated film, non-crimp fabric, a resin mix, or any combination thereof.

8. The method of any preceding claim, wherein the sensor data (134, 218, 722) comprises a component temperature, an unconsolidated composite material (204, 702) temperature, an ambient temperature, a humidity, a roller speed, a speed of the unconsolidated composite material (204, 702), a pressure between a pair of rollers, a gap between the pair of rollers, an unconsolidated composite material (204, 702) thickness, a Fourier transform infrared (FTIR) spectra, spectroscopy data, a material width, a fiber web width, a material areal weight, a fiber areal weight, a resin viscosity, an electrical measurement across a dimension of the unconsolidated composite material (204, 702), a voltage across the dimension of the unconsolidated composite material (204, 702), an electromagnetic measurement across the dimension of the unconsolidated composite material (204, 702), an acoustic measurement across the dimension of the unconsolidated composite material (204, 702), a tension, an image of a prepreg (648, 712, 807) at a location in the composite material manufacturing system (206, 500, 600, 708), or nay combination thereof.

9. The method of any preceding claim, comprising receiving upstream information including reinforcement information, fiber information, reinforcement sizing information, resin information, release liner information, resin film information, or nay combination thereof.

10. The method of any preceding claim, wherein the set of predicted properties (224) comprises a prepreg fiber areal weight, prepreg resin content, prepreg thickness, prepreg infiltration level, a prepreg tack level, resin film areal weight, resin film thickness, release liner areal weight, release liner thickness, a laminate structural property, or any combination thereof.

11. A composite material management system (202) comprising:
a computer system (210) including a composite material manager (132, 212), wherein the composite material manager (132, 212) is programmed to perform the method of any preceding claim.

12. The composite material management system (202) of claim 11, further comprising:
a sensor system (220) including a set of sensors (222, 716) located at a sensor location (660, 662, 664, 666, 667, 669, 671, 718, 812) between a first point (704) and a second point (706) in the composite material manufacturing system (206, 500, 600, 708); and
the first point (704) is a point which a set of actions (241, 710, 720) is performed on a portion (302, 700, 824) of the unconsolidated composite material (204, 702) as the portion (302, 700, 824) of the unconsolidated composite material (204, 702) passes the first point (704).

13. A composite material manufacturing system (206, 500, 600, 708) comprising:
a carbon fiber prepreg coating line that operates to manufacture length of prepreg from fibers and a resin coated film; and
the composite material management system (202) of claim 12;
wherein the sensor system (220) is operable to generate the sensor data (134, 218, 722) for the carbon fiber prepreg coating line during operation of the carbon fiber prepreg coating line.

14. A computer program comprising computer readable program instructions that, when executed by one or more computer processors of a computer system, cause the computer system to perform the method of any of claims 1 to 10.

15. A computer-readable medium having stored thereon the computer program of claim 14.

## Patentansprüche

1. Computerimplementiertes Verfahren zur Herstellung eines unkonsolidierten Verbundmaterials, wobei das Verfahren Folgendes umfasst:
Empfangen von Sensordaten (134, 218, 722) von einem Sensorsystem (220) für ein Verbundmaterialherstellungssystem (206, 500, 600, 708), wobei sich die Sensordaten (134, 218, 722) auf mindestens eines des unkonsolidierten Verbundmaterials (204) oder des Verbundmaterialherstellungssystems (208) während des Betriebs des Verbundmaterialherstellungssystems (208) beziehen und während des Herstellens eines unkonsolidierten Verbundmaterials (204, 702) durch das Verbundmaterialherstellungssystem (206, 500, 600, 708) empfangen werden;
Bestimmung eines Satzes von vorhergesagten Eigenschaften (224) für eine Anzahl von Abschnitten (226) des unkonsolidierten Verbundmaterials (204, 702), wie es vom Verbundmaterialherstellungssystem (206, 500, 600, 708) fertig hergestellt wurde, unter Verwendung der Sensordaten (134, 218, 722) und eines Modells maschinellen Lernens (138, 236), wobei:
das Modell maschinellen Lernens darauf trainiert ist, Eigenschaften (228) für das unkonsolidierte Verbundmaterial (204) unter Verwendung von Sensordaten (218) vorherzusagen,
das Modell maschinellen Lernens (138, 236) unter Verwendung eines Trainingsdatensatzes trainiert wurde, der historische Sensordaten (242) umfasst, die bei vorheriger Herstellung des unkonsolidierten Verbundmaterials (204) generiert wurden, und Etiketten in Form von historischen Eigenschaften (244), und
die historischen Eigenschaften (244) aus Messungen an unkonsolidiertem Verbundmaterial (204) an verschiedenen Stellen im Verbundmaterialherstellungssystem (206) bestimmt werden und Eigenschaften (228) des unkonsolidierten Verbundmaterials (204) einschließen, wenn Herstellen des unkonsolidierten Verbundmaterials (204) abgeschlossen wurde; und
Identifizieren eines Qualitätsniveaus (230) für die Anzahl von Abschnitten (226) des unkonsolidierten Verbundmaterials (204, 702) basierend auf dem Satz von vorhergesagten Eigenschaften (224) für die Anzahl von Abschnitten (226) des unkonsolidierten Verbundmaterials (204, 702).

2. Verfahren nach Anspruch 1, umfassend:
Durchführen einer Markieraktion (300) basierend auf dem für die Anzahl von Abschnitten (226) des unkonsolidierten Verbundmaterials (204, 702) bestimmten Qualitätsniveau (230) und optional:
Markieren von mindestens einigen der Anzahl von Abschnitten (226) des unkonsolidierten Verbundmaterials (204, 702) als unbrauchbar (304) basierend darauf, dass das Qualitätsniveau (230) dieser Abschnitte (302, 700, 824) außerhalb einer Toleranz (312) liegt; und/oder
Markieren von mindestens einigen der Anzahl von Abschnitten (226) des unkonsolidierten Verbundmaterials (204, 702) als verwendbar (306) basierend darauf, dass das Qualitätsniveau (230) für diese Abschnitte (302, 700, 824) innerhalb einer Toleranz (312) liegt.

3. Verfahren nach Anspruch 2, wobei Durchführen der Markieraktion (300) Folgendes umfasst:
Markieren der Anzahl von Abschnitten (226) des unkonsolidierten Verbundmaterials (204, 702) mit dem Qualitätsniveau (230);
und/oder
Aufzeichnen des Qualitätsniveaus (230) für die Anzahl von Abschnitten (226) des unkonsolidierten Verbundmaterials (204, 702).

4. Verfahren nach einem vorstehenden Anspruch, wobei es sich bei dem Verbundmaterialherstellungssystem (206, 500, 600, 708) um eine Kohlenstofffaser-Prepreg-Beschichtungslinie, eine Verstärkungsfaser-Harz-Imprägnierlinie, ein Harzmischprozesssystem, eine Harzfolienherstellungslinie, ein Prepreg-System oder ein Schneidprozesssystem handelt.

5. Verfahren nach einem vorstehenden Anspruch, wobei ein Trainingsdatensatz für das Modell maschinellen Lernens die historischen Sensordaten umfasst, und:
(a) Etiketten in Form von historischen Eigenschaften (244), wobei die historischen Eigenschaften (244) optional aus Messungen an unkonsolidiertem Verbundmaterial (204) an verschiedenen Stellen im Verbundmaterialherstellungssystem (206) bestimmt werden; und optional,
(b) vorgelagerte historische Informationen (245), wobei die vorgelagerten historischen Informationen (245) optional historischen Sensordaten (242) zugeordnet sind, die bei der Herstellung eines unkonsolidierten Verbundmaterials erzeugt wurden, wobei der Herstellungsprozess vorgelagerte historische Informationen einschließt, wobei die vorgelagerten historischen Informationen (245) optional Informationen über Komponenten oder Einstellungen umfassen, die bei der Herstellung unkonsolidierter Verbundmaterialien verwendet wurden.

6. Verfahren nach einem vorstehenden Anspruch, weiter umfassend Bestimmen des Satzes von vorhergesagten Eigenschaften (224) für einen Abschnitt (302, 700, 824) des unkonsolidierten Verbundmaterials (204, 702) unter Verwendung der Sensordaten (134, 218, 722), des Modells maschinellen Lernens (138, 236) und eines physikalischen Modells (239) und optional:
Bestimmen eines ersten Satzes von anfänglich vorhergesagten Eigenschaften (251) für den Abschnitt (302, 700, 824) des unkonsolidierten Verbundmaterials (204, 702) aus dem Modell maschinellen Lernens (138, 236), das unter Verwendung der Sensordaten (134, 218, 722) generiert wurde;
Bestimmen eines zweiten Satzes von anfänglich vorhergesagten Eigenschaften (251) für den Abschnitt (302, 700, 824) des unkonsolidierten Verbundmaterials (204, 702) aus dem physikalischen Modell (239), das unter Verwendung der Sensordaten (134, 218, 722) generiert wurde; und
Bestimmen des Satzes von vorhergesagten Eigenschaften (224) für den Abschnitt (302, 700, 824) des unkonsolidierten Verbundmaterials (204, 702) aus einer Verschmelzung des ersten Satzes von anfänglich vorhergesagten Eigenschaften (251) und des zweiten Satzes von anfänglich vorhergesagten Eigenschaften (251).

7. Verfahren nach einem vorstehenden Anspruch, wobei es sich bei dem unkonsolidierten Verbundmaterial (204, 702) um ein Prepreg (648, 712, 807), ein Prepreg in Luft- und Raumfahrtqualität, ein duroplastisches Prepreg, ein thermoplastisches Prepreg, ein gewebtes Prepreg, ein Faserbündel-Prepreg, ein Bündel-Prepreg, ein unidirektionales Band-Prepreg, eine harzbeschichtete Folie, ein nicht gekräuseltes Gewebe, eine Harzmischung oder eine beliebige Kombination davon handelt.

8. Verfahren nach einem vorstehenden Anspruch, wobei die Sensordaten (134, 218, 722) eine Komponententemperatur, eine Temperatur des unkonsolidierten Verbundmaterials (204, 702), eine Umgebungstemperatur, eine Luftfeuchtigkeit, eine Walzengeschwindigkeit, eine Geschwindigkeit des unkonsolidierten Verbundmaterials (204, 702), einen Druck zwischen einem Walzenpaar, einen Spalt zwischen einem Walzenpaar, eine Dicke des unkonsolidierten Verbundmaterials (204, 702), ein Fourier-Transformations-Infrarot-(FTIR)-Spektrum, Spektroskopiedaten, eine Materialbreite, eine Faserbahnbreite, ein Flächengewicht des Materials, ein Flächengewicht der Fasern, eine Harzviskosität, eine elektrische Messung über eine Dimension des unkonsolidierten Verbundmaterials (204, 702), eine Spannung über eine Dimension des unkonsolidierten Verbundmaterials (204, 702), eine elektromagnetische Messung über eine Dimension des unkonsolidierten Verbundmaterials (204, 702), eine akustische Messung über eine Dimension des unkonsolidierten Verbundmaterials (204, 702), eine Spannung, ein Bild eines Prepregs (648, 712, 807) an einer Stelle im Verbundmaterialherstellungssystem (206, 500, 600, 708) oder eine beliebige Kombination davon umfassen.

9. Verfahren nach einem vorstehenden Anspruch, umfassend Empfangen von vorgelagerten Informationen, die Verstärkungsinformationen, Faserinformationen, Informationen über die Größe von Verstärkung, Harzinformationen, Trägermaterialinformationen, Harzfolieninformationen oder eine beliebige Kombination davon einschließen.

10. Verfahren nach einem vorstehenden Anspruch, wobei der Satz von vorhergesagten Eigenschaften (224) ein Flächengewicht der Prepreg-Fasern, Harzgehalt des Prepregs, Dicke des Prepregs, Infiltrationsgrad des Prepregs, den Klebrigkeitsgrad des Prepregs, Flächengewicht der Harzfolie, Dicke der Harzfolie, Flächengewicht des Trägermaterials, Dicke des Trägermaterials, strukturelle Eigenschaft des Laminats oder eine beliebige Kombination davon umfasst.

11. Verbundmaterialverwaltungssystem (202), umfassend:
ein Computersystem (210), das einen Verbundmaterialverwalter (132, 212) einschließt, wobei der Verbundmaterialverwalter (132, 212) so programmiert ist, dass er das Verfahren nach einem vorstehenden Anspruch durchführt.

12. Verbundmaterialverwaltungssystem (202) nach Anspruch 11, weiter umfassend:
ein Sensorsystem (220), das einen Satz von Sensoren (222, 716) einschließt, die an einer Sensorposition (660, 662, 664, 666, 667, 669, 671, 718, 812) zwischen einem ersten Punkt (704) und einem zweiten Punkt (706) im Verbundmaterialherstellungssystem (206, 500, 600, 708) gelegen sind; und
wobei der erste Punkt (704) ein Punkt ist, an dem ein Satz von Aktionen (241, 710, 720) an einem Abschnitt (302, 700, 824) des unkonsolidierten Verbundmaterials (204, 702) durchgeführt wird, während der Abschnitt (302, 700, 824) des unkonsolidierten Verbundmaterials (204, 702) den ersten Punkt (704) passiert.

13. Verbundmaterialherstellungssystem (206, 500, 600, 708), umfassend:
eine Kohlenstofffaser-Prepreg-Beschichtungslinie, die arbeitet, um Prepreg-Länge aus Fasern und einer harzbeschichteten Folie herzustellen; und
das Verbundmaterialverwaltungssystem (202) nach Anspruch 12;
wobei das Sensorsystem (220) betriebsfähig ist, um die Sensordaten (134, 218, 722) für die Kohlenstofffaser-Prepreg-Beschichtungslinie während des Betriebs der Kohlenstofffaser-Prepreg-Beschichtungslinie zu generieren.

14. Computerprogramm, das computerlesbare Anweisungen umfasst, die, wenn sie von einem oder mehreren Computerprozessoren eines Computersystems ausgeführt werden, bewirken, dass der Computerprozessor das Verfahren nach einem der Ansprüche 1 bis 10 durchführt.

15. Computerlesbares Medium, das darauf das Computerprogramm nach Anspruch 14 gespeichert aufweist.

## Revendications

1. Procédé de fabrication d'un matériau composite non consolidé mis en œuvre par ordinateur, le procédé comprenant :
la réception de données de capteur (134, 218, 722) provenant d'un système de capteurs (220) pour un système de fabrication de matériau composite (206, 500, 600, 708), dans lequel les données de capteur (134, 218, 722) concernent au moins le matériau composite non consolidé (204) ou le système de fabrication de matériau composite (208) pendant le fonctionnement du système de fabrication de matériau composite (208) et sont reçues pendant la fabrication d'un matériau composite non consolidé (204, 702) par le système de fabrication de matériau composite (206, 500, 600, 708) ;
la détermination d'un ensemble de propriétés prédites (224) pour un certain nombre de portions (226) du matériau composite non consolidé (204, 702) tel qu'il est fabriqué par le système de fabrication de matériau composite (206, 500, 600, 708) à l'aide des données de capteur (134, 218, 722) et d'un modèle d'apprentissage automatique (138, 236), dans lequel :
le modèle d'apprentissage automatique est entraîné à prédire les propriétés (228) du matériau composite non consolidé (204) à l'aide de données de capteur (218),
le modèle d'apprentissage automatique (138, 236) a été entraîné à l'aide d'un ensemble de données d'entraînement comprenant des données de capteur historiques (242) générées lors de la fabrication antérieure du matériau composite non consolidé (204) et des étiquettes sous forme de propriétés historiques (244), et
les propriétés historiques (244) sont déterminées à partir de mesures effectuées sur le matériau composite non consolidé (204) à différents endroits du système de fabrication de matériau composite (206), et incluent les propriétés (228) du matériau composite non consolidé (204) lorsque la fabrication du matériau composite non consolidé (204) est terminée ; et
l'identification d'un niveau de qualité (230) pour le nombre de portions (226) du matériau composite non consolidé (204, 702) sur la base de l'ensemble des propriétés prédites (224) pour le nombre de portions (226) du matériau composite non consolidé (204, 702).

2. Procédé selon la revendication 1, comprenant :
la réalisation d'une action de marquage (300) sur la base du niveau de qualité (230) identifié pour le nombre de portions (226) du matériau composite non consolidé (204, 702) et, facultativement :
le marquage d'au moins certaines portions (226) du matériau composite non consolidé (204, 702) comme inutilisables (304) sur la base du niveau de qualité (230) pour ces portions (302, 700, 824) étant hors tolérance (312) ; et/ou
le marquage d'au moins certaines portions (226) du matériau composite non consolidé (204, 702) comme utilisables (306) sur la base du niveau de qualité (230) pour ces portions (302, 700, 824) étant dans une tolérance (312).

3. Procédé selon la revendication 2, dans lequel l'exécution de l'action de marquage (300) comprend :
le marquage du nombre de portions (226) du matériau composite non consolidé (204, 702) avec le niveau de qualité (230) ;
et/ou
l'enregistrement du niveau de qualité (230) pour le nombre de portions (226) du matériau composite non consolidé (204, 702).

4. Procédé selon une quelconque revendication précédente, dans lequel le système de fabrication de matériau composite (206, 500, 600, 708) est une ligne de revêtement de préimprégné de fibres de carbone, une ligne d'imprégnation de résine de fibres de renforcement, un système de processus de mélange de résine, une ligne de filmage de résine, un système de préimprégné ou un système de processus de refendage.

5. Procédé selon une quelconque revendication précédente dans lequel un ensemble de données d'entraînement pour le modèle d'apprentissage automatique comprend les données de capteur historiques, et :
(a) des étiquettes sous forme de propriétés historiques (244), dans lequel, facultativement, les propriétés historiques (244) sont déterminées à partir de mesures effectuées sur un matériau composite non consolidé (204) à différents endroits du système de fabrication du matériau composite (206) ; et, facultativement,
(b) des informations amont historiques (245), dans lequel, facultativement, les informations amont historiques (245) sont associées à des données de capteur historiques (242) générées lors de la fabrication d'un matériau composite non consolidé dans lequel le processus de fabrication inclut des informations amont historiques, dans lequel les informations amont historiques (245) comprennent facultativement des informations sur les composants ou les paramètres utilisés dans la fabrication de matériaux composites non consolidés.

6. Procédé selon une quelconque revendication précédente, comprenant en outre la détermination de l'ensemble des propriétés prédites (224) pour une portion (302, 700, 824) du matériau composite non consolidé (204, 702) à l'aide des données de capteur (134, 218, 722), du modèle d'apprentissage automatique (138, 236) et d'un modèle physique (239) et, facultativement :
la détermination d'un premier ensemble de propriétés initiales prédites (251) pour la portion (302, 700, 824) du matériau composite non consolidé (204, 702) à partir du modèle d'apprentissage automatique (138, 236) généré à l'aide des données de capteur (134, 218, 722) ;
la détermination d'un second ensemble de propriétés initiales prédites (251) pour la portion (302, 700, 824) du matériau composite non consolidé (204, 702) à partir du modèle physique (239) généré à l'aide des données de capteur (134, 218, 722) ; et
la détermination de l'ensemble des propriétés prédites (224) pour la portion (302, 700, 824) du matériau composite non consolidé (204, 702) à partir d'une fusion du premier ensemble de propriétés prédites initiales (251) et du second ensemble de propriétés prédites initiales (251).

7. Procédé selon une quelconque revendication précédente, dans lequel le matériau composite non consolidé (204, 702) est un préimprégné (648, 712, 807), un préimprégné de qualité aérospatiale, un préimprégné thermodurcissable, un préimprégné thermoplastique, un préimprégné de tissu tissé, un préimprégné de mèche de fibres, un préimprégné de mèche, un préimprégné de ruban unidirectionnel, un film enduit de résine, un tissu non-froissé, un mélange de résine ou toute combinaison de ceux-ci.

8. Procédé selon une quelconque revendication précédente, dans lequel les données de capteur (134, 218, 722) comprennent la température de composant, la température de matériau composite non consolidé (204, 702), la température ambiante, l'humidité, la vitesse des rouleaux, la vitesse du matériau composite non consolidé (204, 702), la pression entre deux rouleaux, l'écart entre la paire de rouleaux, l'épaisseur du matériau composite non consolidé (204, 702), le spectre infrarouge à transformée de Fourier (IRTF), des données de spectroscopie, la largeur du matériau, la largeur de fibres, la masse surfacique du matériau, la masse surfacique des fibres, la viscosité de la résine, la mesure électrique aux bornes d'une dimension du matériau composite non consolidé (204, 702), la tension aux bornes d'une dimension du matériau composite non consolidé (204, 702), la mesure électromagnétique aux bornes d'une dimension du matériau composite non consolidé (204, 702), la mesure acoustique aux bornes d'une dimension du matériau composite non consolidé (204, 702), la tension, l'image d'un préimprégné (648, 712, 807) à un emplacement dans le système de fabrication de matériaux composites (206, 500, 600, 708), ou toute combinaison de ceux-ci.

9. Procédé selon une quelconque revendication précédente, comprenant la réception d'informations en amont, incluant des informations sur le renforcement, des informations sur les fibres, des informations sur le dimensionnement du renforcement, des informations sur la résine, des informations sur le revêtement anti-adhésif, des informations sur le film de résine, ou toute combinaison de celles-ci.

10. Procédé selon une quelconque revendication précédente, dans lequel l'ensemble des propriétés prédites (224) comprend une masse surfacique de fibre préimprégnée, une teneur en résine préimprégnée, une épaisseur préimprégnée, un niveau d'infiltration préimprégné, un niveau d'adhérence préimprégné, une masse surfacique de film de résine, une épaisseur de film de résine, une masse surfacique de doublure antiadhésive, une épaisseur de revêtement anti-adhésif, une propriété structurelle de stratifié, ou toute combinaison de ceux-ci.

11. Système de gestion de matériau composite (202), comprenant :
un système informatique (210) incluant un gestionnaire de matériau composite (132, 212), dans lequel le gestionnaire de matériau composite (132, 212) est programmé pour exécuter le procédé selon une quelconque revendication précédente.

12. Système de gestion de matériau composite (202) selon la revendication 11, comprenant en outre :
un système de capteur (220) incluant un ensemble de capteurs (222, 716) situés à un emplacement de capteur (660, 662, 664, 666, 667, 669, 671, 718, 812) entre un premier point (704) et un second point (706) dans le système de fabrication de matériau composite (206, 500, 600, 708) ; et
le premier point (704) est un point sur lequel un ensemble d'actions (241, 710, 720) est effectué sur une portion (302, 700, 824) du matériau composite non consolidé (204, 702) lorsque la portion (302, 700, 824) du matériau composite non consolidé (204, 702) passe par le premier point (704).

13. Système de fabrication de matériau composite (206, 500, 600, 708) comprenant :
une ligne de revêtement de préimprégné en fibre de carbone qui permet de fabriquer des longueurs de préimprégné à partir de fibres et d'un film enduit de résine ; et
le système de gestion de matériaux composite (202) selon la revendication 12 ;
dans lequel le système de capteur (220) est opérationnel pour générer les données de capteur (134, 218, 722) pour la ligne de revêtement de préimprégné de fibres de carbone pendant le fonctionnement de la ligne de revêtement de préimprégné de fibres de carbone.

14. Programme informatique comprenant des instructions de programme lisibles par ordinateur qui, lorsqu'elles sont exécutées par un ou plusieurs processeurs d'un système informatique, amènent le système informatique à exécuter le procédé selon l'une quelconque des revendications 1 à 10.

15. Support lisible par ordinateur sur lequel est stocké le programme informatique selon la revendication 14.
